# EUROPEAN PATENT SPECIFICATION

(11) **EP 3 564 390 B1**
(45) Date of publication and mention of the grant of the patent: **29.11.2023**
(21) Application number: 17887675.1
(22) Date of filing: 27.12.2017
(51) Int. Cl.: C12Q 1/689

(54) **METHOD FOR DIAGNOSING COLON TUMOR VIA BACTERIAL METAGENOMIC ANALYSIS**
VERFAHREN ZUR DIAGNOSE VON KOLONTUMOREN DURCH BAKTERIELLE METAGENOME ANALYSE
PROCÉDÉ DE DIAGNOSTIC D'UNE TUMEUR AU CÔLON PAR L'INTERMÉDIAIRE D'UNE ANALYSE MÉTAGÉNOMIQUE BACTÉRIENNE

(30) Priority: 28.12.2016 KR 20160181574; 26.12.2017 KR 20170180144
(43) Date of publication of application: 06.11.2019
(73) Proprietor: MD Healthcare Inc., Seoul 03923 (KR)
(72) Inventor: KIM, Yoon-Keun, Namyangju-si Gyeonggi-do 12146 (KR)
(74) Representative: Dehns
(86) International application number: PCT/KR2017/015557
(87) International publication number: WO 2018/124735

(56) References cited:
- EP-A1- 2 955 232
- WO-A1-2016/085356
- WO-A1-2016/099076
- WO-A1-2017/062625
- WO-A1-2019/004668
- KR-A- 20110 025 603
- KR-A- 20160 110 232
- US-A1- 2013 121 968
- BARTENEVA NATASHA S ET AL: "Extracellular vesicles in gastrointestinal cancer in conjunction with microbiota: On the border of Kingdoms", BBA - REVIEWS ON CANCER, vol. 1868, no. 2, 29 June 2017 (2017-06-29), pages 372-393, XP085255158, ISSN: 0304-419X, DOI: 10.1016/J.BBCAN.2017.06.005
- J. P. ZACKULAR ET AL: "The Human Gut Microbiome as a Screening Tool for Colorectal Cancer", CANCER PREVENTION RESEARCH, vol. 7, no. 11, 7 August 2014 (2014-08-07) , pages 1112-1121, XP055333767, United States ISSN: 1940-6207, DOI: 10.1158/1940-6207.CAPR-14-0129
- Zeller, George et al: "Potential of fecal microbiota for early-stage detection of Colorectal Cancer", Molecular Systems Biology, vol. 10, no. 11, 28 November 2014 (2014-11-28), page 766, XP055488670, DOI: 10.15252/msb.20145645

## Description

### [Technical Field]

The present invention relates to a method of diagnosing colon tumors through bacterial metagenomic analysis, and more particularly, to a method of diagnosing colon tumors such as colonic polyps, colon cancer, and the like by analyzing an increase or decrease in content of extracellular vesicles derived from specific bacteria through bacterial metagenomic analysis using a subject-derived sample.

### [Background Art]

Colon cancer or colorectal cancer is a malignant tumor occurring in the appendix, colon, and rectum and occurs in the mucous membrane, which is the innermost surface of the large intestine. According to an announcement in 2006, colon cancer is the second most common cancer after gastric cancer in Korea, and the frequency of onset thereof has sharply increased as eating habits have recently been westernized, and in the last decade, mortality from colon cancer has increased by about 80% and the rate of increase is continuously increasing. Colon cancer occurs most frequently in those in their 60s, and as for an occurrence site, colon cancer occurs more frequently in the rectum than in the colon. Although colon cancer may occur at any age, 90% or more of patients with colon cancer are 40 years old or older, and the incidence rate has doubled every 10 years.

All colon cancers are known to begin with a colon polyp or colon adenoma, and a polyp refers to a protrusion occurring by initial abnormal growth of the epithelium in the intestinal lining, and is a very common disease occurring in 15% to 20% of adults. In addition to colon polyps, if you have a family member with colon cancer or you are an individual suffering from ulcerative colitis for a long period of time, the risk of colon cancer increases. In addition, colon cancer is a typical cancer known to be associated with foods, and as causative factors of colon cancer, low intake of cellulose due to westernization of dietary life, high intake of animal fats, excess intake of refined sugar, and the like are known.

In the case of early colon cancer, there is no specific symptom, but even if there is no symptom, unnoticed intestinal bleeding occurs and thus blood is lost, resulting in the occurrence of anemia, and sometimes, a lack of appetite and weight loss may occur. When cancer progresses, a stomachache or a change in bowel habits, such as diarrhea, constipation, or the like may occur, or there may be symptoms of rectal bleeding from the anus, and blood may have a bright scarlet color or a black color. When colon cancer is developed, a mass that has not been usually recognized in the abdomen may be felt. The most notable symptoms are a change in bowel habits, bloody stool, pain, and anemia, and particularly, it is necessary to thoroughly investigate such changes in adults aged 40 or more years old.

The definite diagnosis of colon cancer is possible when cancer cells are detected through a biopsy by colonoscopy. In most colon cancer cases, there is no early symptom so that diagnosis is very difficult, and there is currently no method of predicting colon cancer using a non-invasive method. There are many cases in which solid cancers such as colon cancer and the like are detected using an existing diagnosis method after cancer development, and thus to reduce medical costs and prevent death due to colon cancer, it is effective to provide a method of preventing the onset of colon cancer in a high risk group by predicting the occurrence of colon tumor and causative factors.

Meanwhile, it is known that the number of microorganisms symbiotically living in the human body is 100 trillion which is 10 times the number of human cells, and the number of genes of microorganisms exceeds 100 times the number of human genes. A microbiota or microbiome is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment (Korean Patent Publication No. 2011-0073049). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, in the onset of colon tumors, there has been no report of a method of diagnosing colon tumors by isolating bacteria and bacteria-derived vesicles from a human-derived substance such as stool and identifying causative factors of colon tumors such as colon polyps, colon cancer, and the like through analysis of metagenomes present in the bacteria-derived vesicles.

WO 2019/004668 discloses a vesicle derived from *Proteus* genus bacteria, and methods and compositions for preventing, treating, and/or improving cancer, cardiovascular diseases, metabolic diseases, neuropsychiatric diseases, allergic respiratory diseases, and inflammatory intestinal diseases.

Barteneva NS, et al. Extracellular vesicles in gastrointestinal cancer in conjunction with microbiota: On the border of Kingdoms. Biochim Biophys Acta Rev Cancer. 2017; 1868(2):372-393, discloses research evaluating the role of EV production in gastrointestinal (GI) cancer development in conjunction with GI microbiota and inflammatory diseases.

EP 2955232 discloses a method for diagnosing adenomas and/or colorectal cancer (CRC) based on analysing the gut microbiome.

WO 2016/085356 discloses compositions prepared from gold kiwifruit, Actinidia chinemis, methods of preparing such compositions, and methods of using such compositions, including methods of treating or preventing disorders of the gastrointestinal system.

WO 2017/062625 discloses a method and kit to detect colon cancer in a human by detecting the presence or absence, or relative amount (abundance) of, certain bacteria in a sample.

Zackular JP, et al. The human gut microbiome as a screening tool for colorectal cancer. Cancer Prev Res (Phila). 2014;7(11): 1112-1121, discloses the feasibility of using the composition of the gut microbiome to detect the presence of precancerous and cancerous lesions.

Zeller G, et al. Mol Syst Biol. 2014;10(11):766. Published 2014 Nov 28, discloses the potential of fecal microbiota for early-stage detection of colorectal cancer.

### [Disclosure]

### [Technical Problem]

To diagnose colon tumors such as colon polyps, colon cancer, and the like, the inventors of the present invention extracted DNA from bacteria-derived extracellular vesicles using urine and stool, which is a subject-derived sample, and performed metagenomic analysis on the extracted DNA, and, as a result, identified bacteria-derived extracellular vesicles capable of acting as a causative factor of colon tumors such as colon polyps, colon cancer, and the like.

Therefore, it is an object of the present invention to provide a method of providing information for colon tumor diagnosis by metagenomic analysis of genes present in bacteria-derived extracellular vesicles.

However, the technical goals of the present invention are not limited to the aforementioned goals, and other unmentioned technical goals will be clearly understood by those of ordinary skill in the art from the following description.

### [Technical Solution]

To achieve the above-described object of the present invention, the present invention provides a method as defined in the appended claims. The invention provides a method of diagnosing colon tumor, comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles to a normal individual-derived sample through sequencing of a product of the PCR,
wherein, in process (c), colon cancer is diagnosed, wherein the comparing comprises comparing an increase or decrease in content of:
extracellular vesicles derived from one or more bacteria selected from the group consisting of the phylum *Tenericutes,* the phylum *Actinobacteria,* the phylum *Acidobacteria,* the phylum *Armatimonadetes,* the phylum *Planctomycetes,* the phylum *Fusobacteria,* the phylum *Proteobacteria,* and the phylum *Euryarchaeota;*
extracellular vesicles derived from one or more bacteria selected from the group consisting of the class *Deferribacteres,* the class *Mollicutes,* the class *Bacilli,* the class *Alphaproteobacteria,* the class *Saprospirae,* the class *Fimbriimonadia,* the class *Acidobacteria-6,* the class *Solibacteres,* the class *Coriobacteriia,* the class *Oscillatoriophycideae,* the class *Fusobacteriia,* the class *Gammaproteobacteria,* the class *Clostridia,* and the class *Methanobacteria*;
extracellular vesicles derived from one or more bacteria selected from the group consisting of the order *Deferribacterales,* the order *Turicibacterales,* the order *Oceanospirillales,* the order *Rhizobiales,* the order *Lactobacillales,* the order *Rhodobacterales,* the order *Saprospirales,* the order *Sphingomonadales,* the order *Fimbriimonadales,* the order *Solibacterales,* the order *Coriobacteriales,* the order *Chroococcales,* the order *Fusobacteriales,* the order *Bdellovibrionales,* the order *Desulfobacterales,* the order *Stramenopiles,* the order *Pseudomonadales,* the order *Desulfovibrionales,* and the order *Methanobacteriales;*
extracellular vesicles derived from one or more bacteria selected from the group consisting of the family *Peptococcaceae,* the family *Deferribacteraceae,* the family *Turicibacteraceae,* the family *Halomonadaceae,* the family *Clostridiaceae,* the family *Prevotellaceae,* the family *Peptostreptococcaceae,* the family *Rhodobacteraceae,* the family *Nocardioidaceae,* the family *Sphingomonadaceae,* the family *Bartonellaceae,* the family *Cellulomonadaceae,* the family *Lactobacillaceae,* the family *Rhizobiaceae,* the family *Fimbriimonadaceae,* the family *Dermacoccaceae,* the family *Leptotrichiaceae,* the family *Coriobacteriaceae,* the family *Xenococcaceae,* the family *Aeromonadaceae,* the family *Geodermatophilaceae,* the family *Bdellovibrionaceae,* the family *Moraxellaceae,* the family *Pseudomonadaceae,* the family *Streptococcaceae,* the family *Veillonellaceae,* the family *Bacteroidaceae,* the family *Aerococcaceae,* the family *Comamonadaceae,* the family *Paraprevotellaceae,* the family *Christensenellaceae,* the family *Ruminococcaceae,* the family *Corynebacteriaceae,* the family *Gordoniaceae,* the family *Mycobacteriaceae,* the family *Desulfovibrionaceae,* the family *Alcaligenaceae,* the family *Barnesiellaceae,* the family *Methanobacteriaceae,* and the family *Rikenellaceae;* or
extracellular vesicles derived from one or more bacteria selected from the group consisting of the genus *Catenibacterium,* the genus *Mucispirillum,* the genus *Eubacterium,* the genus *Turicibacter,* the genus *Alloiococcus,* the genus *Halomonas,* the genus *Prevotella,* the genus *Dialister,* the genus *Anaerostipes,* the genus *Faecalibacterium,* the genus *Blautia,* the genus *Capnocytophaga,* the genus *Sphingomonas,* the genus *Lactobacillus,* the genus *Fimbriimonas,* the genus *Dermacoccus,* the genus *Achromobacter,* the genus *Novosphingobium,* the genus *Sneathia,* the genus *Agrobacterium,* the genus *Blastomonas,* the genus *Bdellovibrio,* the genus *Alkanindiges,* the genus *Roseateles,* the genus *Shuttleworthia,* the genus *Rhizobium,* the genus *Morganella,* the genus *Acinetobacter,* the genus *Pseudomonas,* the genus *Enterococcus,* the genus *Lactococcus,* the genus *Coprococcus,* the genus *Dorea,* the genus *Streptococcus,* the genus *Lachnospira,* the genus *Ruminococcus,* the genus *Corynebacterium,* the genus *Comamonas,* the genus *Gordonia,* the genus *Paraprevotella,* the genus *Mycobacterium,* the genus *Roseburia,* the genus *Slackia,* the genus *Escherichia,* the genus *Phascolarctobacterium,* the genus *Sutterella,* the genus *Virgibacillus,* the genus *Eggerthella,* the genus *Citrobacter,* the genus *Roseomonas,* the genus *Serratia,* the genus *Methanobrevibacter,* and the genus *Bilophila,* or
wherein, in process (c), colon polyp may be diagnosed, wherein the comparing may comprise comparing an increase or decrease in content of:
   extracellular vesicles derived from one or more bacteria selected from the group consisting of the phylum *Proteobacteria,* and the phylum *Euryarchaeota;*
   extracellular vesicles derived from one or more bacteria selected from the group consisting of the class *Gammaproteobacteria,* the class *Clostridia,* and the class *Methanobacteria*;
   extracellular vesicles derived from one or more bacteria selected from the group consisting of the order *Stramenopiles,* the order *Pseudomonadales,* the order *Oceanospirillales,* the order *Desulfovibrionales,* and the order *Methanobacteriales;*
   extracellular vesicles derived from one or more bacteria selected from the group consisting of the family *Rhizobiaceae,* the family *Exiguobacteraceae,* the family *Moraxellaceae,* the family *Pseudomonadaceae,* the family *Streptococcaceae,* the family *Peptostreptococcaceae,* the family *Comamonadaceae,* the family *Veillonellaceae,* the family *Bacteroidaceae,* the family *Paraprevotellaceae,* the family *Ruminococcaceae,* the family *Corynebacteriaceae,* the family *Christensenellaceae,* the family *Odoribacteraceae,* the family *Desulfovibrionaceae,* the family *Halomonadaceae,* the family *Alcaligenaceae,* the family *Barnesiellaceae,* the family *Methanobacteriaceae,* and the family *Rikenellaceae;* or
   extracellular vesicles derived from one or more bacteria selected from the group consisting of the genus *Rhizobium,* the genus *Morganella,* the genus *Exiguobacterium,* the genus *Acinetobacter,* the genus *Pseudomonas,* the genus *Lactococcus,* the genus *Coprococcus,* the genus *Streptococcus,* the genus *Ruminococcus,* the genus *Corynebacterium,* the genus *Odoribacter,* the genus *Clostridium,* the genus *Comamonas,* the genus *Paraprevotella,* the genus *Roseburia,* the genus *Citrobacter,* the genus *Klebsiella,* the genus *Virgibacillus,* the genus *Slackia,* the genus *Dialister,* the genus *Phascolarctobacterium,* the genus *Sutterella,* the genus *Halomonas,* the genus *Roseomonas,* and the genus *Methanobrevibacter.*

In one embodiment of the present invention, the subject sample may be stool or urine.

### [Advantageous Effects]

Extracellular vesicles secreted from bacteria present in the environment are absorbed into the human body, and thus may directly affect the occurrence of inflammation and cancer, and it is difficult to diagnose colon polyp and colon cancer early before symptoms occur, and thus efficient treatment therefor is difficult. Thus, a risk of developing colon tumors such as colon polyps, colon cancer, and the like can be predicted through metagenomic analysis of bacteria or bacteria-derived extracellular vesicles by using a human body-derived sample, and thus the onset of colon tumor can be delayed or colon tumor can be prevented through appropriate management by early diagnosis and prediction of a risk group for colon tumor, and, even after colon tumor occur, early diagnosis for colon tumor can be implemented, thereby lowering the incidence rate of colon tumor and increasing therapeutic effects. In addition, patients diagnosed with a colon polyp or colon cancer can avoid exposure to causative factors predicted by metagenomic analysis, whereby the progression of a colon polyp and colon cancer can be ameliorated, or recurrence thereof can be prevented.

### [Description of Drawings]

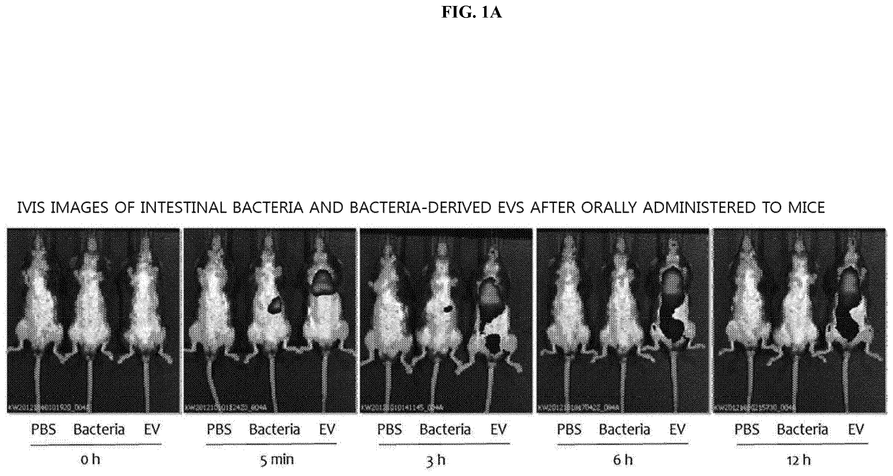

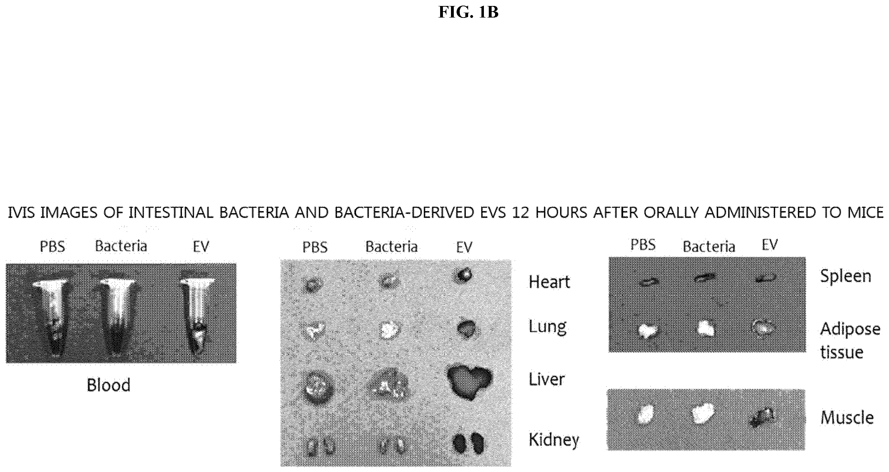

FIGS. 1A and 1B are views for evaluating the distribution pattern of extracellular vesicles (EVs) derived from bacteria *in vivo.* FIG. 1A illustrates images showing the distribution pattern of intestinal bacteria and EVs derived from bacteria per time (0 h, 5 min, 3 h, 6 h, and 12 h) after being orally administered to mice. FIG. 1B illustrates images showing the distribution pattern of gut bacteria and EVs derived from bacteria after being orally administered to mice and, after 12 hours, blood and various organs (heart, lung, liver, kidney, spleen, adipose tissue, and muscle) of the mice were extracted.
FIG. 2 illustrates distribution results of bacteria-derived EVs exhibiting significant diagnostic performance at a phylum level, after metagenomic analysis of bacteria-derived EVs isolated from colon cancer patient-derived stool and normal individual-derived stool.
FIG. 3 illustrates distribution results of bacteria-derived EVs exhibiting significant diagnostic performance at a class level, after metagenomic analysis of bacteria-derived EVs isolated from colon cancer patient-derived stool and normal individual-derived stool.
FIG. 4 illustrates distribution results of bacteria-derived EVs exhibiting significant diagnostic performance at an order level, after metagenomic analysis of bacteria-derived EVs isolated from colon cancer patient-derived stool and normal individual-derived stool.
FIG. 5 illustrates distribution results of bacteria-derived EVs exhibiting significant diagnostic performance at a family level, after metagenomic analysis of bacteria-derived EVs isolated from colon cancer patient-derived stool and normal individual-derived stool.
FIG. 6 illustrates distribution results of bacteria-derived EVs exhibiting significant diagnostic performance at a genus level, after metagenomic analysis of bacteria-derived EVs isolated from colon cancer patient-derived stool and normal individual-derived stool.
FIG. 7 illustrates distribution results of bacteria-derived EVs exhibiting significant diagnostic performance at a phylum level, after metagenomic analysis of bacteria-derived EVs isolated from colon cancer patient-derived urine and normal individual-derived urine.
FIG. 8 illustrates distribution results of bacteria-derived EVs exhibiting significant diagnostic performance at a class level, after metagenomic analysis of bacteria-derived EVs isolated from colon cancer patient-derived urine and normal individual-derived urine.
FIG. 9 illustrates distribution results of bacteria-derived EVs exhibiting significant diagnostic performance at an order level, after metagenomic analysis of bacteria-derived EVs isolated from colon cancer patient-derived urine and normal individual-derived urine.
FIG. 10 illustrates distribution results of bacteria-derived EVs exhibiting significant diagnostic performance at a family level, after metagenomic analysis of bacteria-derived EVs isolated from colon cancer patient-derived urine and normal individual-derived urine.
FIG. 11 illustrates distribution results of bacteria-derived EVs exhibiting significant diagnostic performance at a genus level, after metagenomic analysis of bacteria-derived EVs isolated from colon cancer patient-derived urine and normal individual-derived urine.
FIG. 12 illustrates distribution results of bacteria-derived EVs exhibiting significant diagnostic performance at a phylum level, after metagenomic analysis of bacteria-derived EVs isolated from colon cancer patient-derived stool and colon polyp patient-derived stool.
FIG. 13 illustrates distribution results of bacteria-derived EVs exhibiting significant diagnostic performance at a class level, after metagenomic analysis of bacteria-derived EVs isolated from colon cancer patient-derived stool and colon polyp patient-derived stool.
FIG. 14 illustrates distribution results of bacteria-derived EVs exhibiting significant diagnostic performance at an order level, after metagenomic analysis of bacteria-derived EVs isolated from colon cancer patient-derived stool and colon polyp patient-derived stool.
FIG. 15 illustrates distribution results of bacteria-derived EVs exhibiting significant diagnostic performance at a family level, after metagenomic analysis of bacteria-derived EVs isolated from colon cancer patient-derived stool and colon polyp patient-derived stool.
FIG. 16 illustrates distribution results of bacteria-derived EVs exhibiting significant diagnostic performance at a genus level, after metagenomic analysis of bacteria-derived EVs isolated from colon cancer patient-derived stool and colon polyp patient-derived stool.
FIG. 17 illustrates distribution results of bacteria-derived EVs exhibiting significant diagnostic performance at an order level, after metagenomic analysis of bacteria-derived EVs isolated from colon cancer patient-derived urine and colon polyp patient-derived urine.
FIG. 18 illustrates distribution results of bacteria-derived EVs exhibiting significant diagnostic performance at a genus level, after metagenomic analysis of bacteria-derived EVs isolated from colon cancer patient-derived urine and colon polyp patient-derived urine.
FIG. 19 illustrates distribution results of bacteria-derived EVs exhibiting significant diagnostic performance at a phylum level, after metagenomic analysis of bacteria-derived EVs isolated from colon polyp patient-derived stool and normal individual-derived stool.
FIG. 20 illustrates distribution results of bacteria-derived EVs exhibiting significant diagnostic performance at a class level, after metagenomic analysis of bacteria-derived EVs isolated from colon polyp patient-derived stool and normal individual-derived stool.
FIG. 21 illustrates distribution results of bacteria-derived EVs exhibiting significant diagnostic performance at an order level, after metagenomic analysis of bacteria-derived EVs isolated from colon polyp patient-derived stool and normal individual-derived stool.
FIG. 22 illustrates distribution results of bacteria-derived EVs exhibiting significant diagnostic performance at a family level, after metagenomic analysis of bacteria-derived EVs isolated from colon polyp patient-derived stool and normal individual-derived stool.
FIG. 23 illustrates distribution results of bacteria-derived EVs exhibiting significant diagnostic performance at a genus level, after metagenomic analysis of bacteria-derived EVs isolated from colon polyp patient-derived stool and normal individual-derived stool.
FIG. 24 illustrates distribution results of bacteria-derived EVs exhibiting significant diagnostic performance at a phylum level, after metagenomic analysis of bacteria-derived EVs isolated from colon polyp patient-derived urine and normal individual-derived urine.
FIG. 25 illustrates distribution results of bacteria-derived EVs exhibiting significant diagnostic performance at a class level, after metagenomic analysis of bacteria-derived EVs isolated from colon polyp patient-derived urine and normal individual-derived urine.
FIG. 26 illustrates distribution results of bacteria-derived EVs exhibiting significant diagnostic performance at an order level, after metagenomic analysis of bacteria-derived EVs isolated from colon polyp patient-derived urine and normal individual-derived urine.
FIG. 27 illustrates distribution results of bacteria-derived EVs exhibiting significant diagnostic performance at a family level, after metagenomic analysis of bacteria-derived EVs isolated from colon polyp patient-derived urine and normal individual-derived urine.
FIG. 28 illustrates distribution results of bacteria-derived EVs exhibiting significant diagnostic performance at a genus level, after metagenomic analysis of bacteria-derived EVs isolated from colon polyp patient-derived urine and normal individual-derived urine.

### [Best Mode]

The present invention relates to a method of diagnosing colon tumors such as colon polyps, colon cancer, and the like through bacterial metagenomic analysis. The inventors of the present invention extracted genes from bacteria-derived extracellular vesicles present in subject-derived samples, performed metagenomic analysis thereon, and identified bacteria-derived extracellular vesicles capable of acting as a causative factor of colon tumors such as colon polyps, colon cancer, and the like.

Thus, the present invention provides a method of diagnosing colon tumor, comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles to a normal individual-derived sample through sequencing of a product of the PCR,
wherein, in process (c), colon cancer is diagnosed, wherein the comparing comprises comparing an increase or decrease in content of:
extracellular vesicles derived from one or more bacteria selected from the group consisting of the phylum *Tenericutes,* the phylum *Actinobacteria,* the phylum *Acidobacteria,* the phylum *Armatimonadetes,* the phylum *Planctomycetes,* the phylum *Fusobacteria,* the phylum *Proteobacteria,* the phylum *Euryarchaeota;*
the class *Deferribacteres,* the class *Mollicutes,* the class *Bacilli,* the class *Alphaproteobacteria,* the class *Saprospirae,* the class *Fimbriimonadia,* the class *Acidobacteria-6,* the class *Solibacteres,* the class *Coriobacteriia,* the class *Oscillatoriophycideae,* the class *Fusobacteriia,* the class *Gammaproteobacteria,* the class *Clostridia,* the class *Methanobacteria*;
the order *Deferribacterales,* the order *Turicibacterales,* the order *Oceanospirillales,* the order *Rhizobiales,* the order *Lactobacillales,* the order *Rhodobacterales,* the order *Saprospirales,* the order *Sphingomonadales,* the order *Fimbriimonadales,* the order *Solibacterales,* the order *Coriobacteriales,* the order *Chroococcales,* the order *Fusobacteriales,* the order *Bdellovibrionales,* the order *Desulfobacterales,* the order *Stramenopiles,* the order *Pseudomonadales,* the order *Desulfovibrionales,* the order *Methanobacteriales;*
the family *Peptococcaceae,* the family *Deferribacteraceae,* the family *Turicibacteraceae,* the family *Halomonadaceae,* the family *Clostridiaceae,* the family *Prevotellaceae,* the family *Peptostreptococcaceae,* the family *Rhodobacteraceae,* the family *Nocardioidaceae,* the family *Sphingomonadaceae,* the family *Bartonellaceae,* the family *Cellulomonadaceae,* the family *Lactobacillaceae,* the family *Rhizobiaceae,* the family *Fimbriimonadaceae,* the family *Dermacoccaceae,* the family *Leptotrichiaceae,* the family *Coriobacteriaceae,* the family *Xenococcaceae,* the family *Aeromonadaceae,* the family *Geodermatophilaceae,* the family *Bdellovibrionaceae,* the family *Moraxellaceae,* the family *Pseudomonadaceae,* the family *Streptococcaceae,* the family *Veillonellaceae,* the family *Bacteroidaceae,* the family *Aerococcaceae,* the family *Comamonadaceae,* the family *Paraprevotellaceae,* the family *Christensenellaceae,* the family *Ruminococcaceae,* the family *Corynebacteriaceae,* the family *Gordoniaceae,* the family *Mycobacteriaceae,* the family *Desulfovibrionaceae,* the family *Alcaligenaceae,* the family *Barnesiellaceae,* the family *Methanobacteriaceae,* the family *Rikenellaceae;*
the genus *Catenibacterium,* the genus *Mucispirillum,* the genus *Eubacterium,* the genus *Turicibacter,* the genus *Alloiococcus,* the genus *Halomonas,* the genus *Prevotella,* the genus *Dialister,* the genus *Anaerostipes,* the genus *Faecalibacterium,* the genus *Blautia,* the genus *Capnocytophaga,* the genus *Sphingomonas,* the genus *Lactobacillus,* the genus *Fimbriimonas,* the genus *Dermacoccus,* the genus *Achromobacter,* the genus *Novosphingobium,* the genus *Sneathia,* the genus *Agrobacterium,* the genus *Blastomonas,* the genus *Bdellovibrio,* the genus *Alkanindiges,* the genus *Roseateles,* the genus *Shuttleworthia,* the genus *Rhizobium,* the genus *Morganella,* the genus *Acinetobacter,* the genus *Pseudomonas,* the genus *Enterococcus,* the genus *Lactococcus,* the genus *Coprococcus,* the genus *Dorea,* the genus *Streptococcus,* the genus *Lachnospira,* the genus *Ruminococcus,* the genus *Corynebacterium,* the genus *Comamonas,* the genus *Gordonia,* the genus *Paraprevotella,* the genus *Mycobacterium,* the genus *Roseburia,* the genus *Slackia,* the genus *Escherichia,* the genus *Phascolarctobacterium,* the genus *Sutterella,* the genus *Virgibacillus,* the genus *Eggerthella,* the genus *Citrobacter,* the genus *Roseomonas,* the genus *Serratia,* the genus *Methanobrevibacter,* and the genus *Bilophila*; or
wherein, in process (c), colon polyp is diagnosed, wherein the comparing comprises comparing an increase or decrease in content of:
   extracellular vesicles derived from one or more bacteria selected from the group consisting of the phylum *Proteobacteria,* the phylum *Euryarchaeota;*
   the class *Gammaproteobacteria,* the class *Clostridia,* the class *Methanobacteria*;
   the order *Stramenopiles,* the order *Pseudomonadales,* the order *Oceanospirillales,* the order *Desulfovibrionales,* the order *Methanobacteriales;*
   the family *Rhizobiaceae,* the family *Exiguobacteraceae,* the family *Moraxellaceae,* the family *Pseudomonadaceae,* the family *Streptococcaceae,* the family *Peptostreptococcaceae,* the family *Comamonadaceae,* the family *Veillonellaceae,* the family *Bacteroidaceae,* the family *Paraprevotellaceae,* the family *Ruminococcaceae,* the family *Corynebacteriaceae,* the family *Christensenellaceae,* the family *Odoribacteraceae,* the family *Desulfovibrionaceae,* the family *Halomonadaceae,* the family *Alcaligenaceae,* the family *Barnesiellaceae,* the family *Methanobacteriaceae,* the family *Rikenellaceae;*
   the genus *Rhizobium,* the genus *Morganella,* the genus *Exiguobacterium,* the genus *Acinetobacter,* the genus *Pseudomonas,* the genus *Lactococcus,* the genus *Coprococcus,* the genus *Streptococcus,* the genus *Ruminococcus,* the genus *Corynebacterium,* the genus *Odoribacter,* the genus *Clostridium,* the genus *Comamonas,* the genus *Paraprevotella,* the genus *Roseburia,* the genus *Citrobacter,* the genus *Klebsiella,* the genus *Virgibacillus,* the genus *Slackia,* the genus *Dialister,* the genus *Phascolarctobacterium,* the genus *Sutterella,* the genus *Halomonas,* the genus *Roseomonas,* and the genus *Methanobrevibacter.*

The term "colon cancer diagnosis" as used herein refers to determining whether a patient has a risk for colon cancer, whether the risk for colon cancer is relatively high, or whether colon cancer has already occurred. The method of the present invention may be used to delay the onset of colon cancer through special and appropriate care for a specific patient, which is a patient having a high risk for colon cancer or prevent the onset of colon cancer. In addition, the method may be clinically used to determine treatment by selecting the most appropriate treatment method through early diagnosis of colon cancer.

The term "colon polyp diagnosis" as used herein refers to determining whether a patient has a risk for colon polyp, whether the risk for colon polyp is relatively high, or whether colon polyp has already occurred. The method of the present invention may be used to delay the onset of colon polyp through special and appropriate care for a specific patient, which is a patient having a high risk for colon polyp or prevent the onset of colon polyp. In addition, the method may be clinically used to determine treatment by selecting the most appropriate treatment method through early diagnosis of colon polyp.

The term "metagenome" as used herein refers to the total of genomes including all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, including genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

Metagenomic analysis was performed on genes present in bacteria-derived extracellular vesicles in stool and urine samples of normal individuals, colon polyp patients, and colon cancer patients, and bacteria-derived extracellular vesicles capable of acting as causes of the onset of colon cancer and a colon polyp were actually identified by analysis at phylum, class, order, family, and genus levels.

As a result of performing metagenomic analysis on bacteria-derived extracellular vesicles present in stool at a phylum level, the content of extracellular vesicles derived from bacteria belonging to the phylum *Deferribacteres,* the phylum *Tenericutes,* the phylum *Actinobacteria,* the phylum *Acidobacteria,* the phylum *Armatimonadetes,* the phylum *Planctomycetes,* and the phylum *Fusobacteria* was significantly different between colon cancer patients and normal individuals (see Example 4).

As a result of performing metagenomic analysis on bacteria-derived extracellular vesicles present in stool at a class level, the content of extracellular vesicles derived from bacteria belonging to the class *Deferribacteres,* the class *Mollicutes,* the class 4C0d-2, the class *Bacilli,* the class *Alphaproteobacteria,* the class *Saprospirae,* the class *Fimbriimonadia,* the class *Acidobacteria-6,* the class *Solibacteres,* the class *Coriobacteriia,* the class *Oscillatoriophycideae,* and the class *Fusobacteriia* was significantly different between colon cancer patients and normal individuals (see Example 4).

As a result of performing metagenomic analysis on bacteria-derived extracellular vesicles present in stool at an order level, the content of extracellular vesicles derived from bacteria belonging to the order RF32, the order YS2, the order *Deferribacterales,* the order *Turicibacterales,* the order RF39, the order *Oceanospirillales,* the order *Rhizobiales,* the order *Lactobacillales,* the order *Rhodobacterales,* the order *Saprospirales,* the order *Sphingomonadales,* the order *Fimbriimonadales,* the order iii1-15, the order *Solibacterales,* the order *Coriobacteriales,* the order *Chroococcales,* the order *Fusobacteriales,* and the order *Bdellovibrionales* was significantly different between colon cancer patients and normal individuals (see Example 4).

As a result of performing metagenomic analysis on bacteria-derived extracellular vesicles present in stool at a family level, the content of extracellular vesicles derived from bacteria belonging to the family *Peptococcaceae,* the family *Deferribacteraceae,* the family *Turicibacteraceae,* the family *Halomonadaceae,* the family *Clostridiaceae,* the family *Prevotellaceae,* the family *Peptostreptococcaceae,* the family *Rhodobacteraceae,* the family *Nocardioidaceae,* the family *Sphingomonadaceae,* the family *Bartonellaceae,* the family *Cellulomonadaceae,* the family *Lactobacillaceae,* the family *Rhizobiaceae,* the family *Fimbriimonadaceae,* the family *Dermacoccaceae,* the family *Leptotrichiaceae,* the family *Coriobacteriaceae,* the family *Xenococcaceae,* the family *Aeromonadaceae,* the family *Geodermatophilaceae,* and the family *Bdellovibrionaceae* was significantly different between colon cancer patients and normal individuals (see Example 4).

As a result of performing metagenomic analysis on bacteria-derived extracellular vesicles present in stool at a genus level, the content of extracellular vesicles derived from bacteria belonging to the genus rc4-4, the genus *Proteus,* the genus *Catenibacterium,* the genus *Mucispirillum,* the genus *Eubacterium,* the genus *Turicibacter,* the genus *Alloiococcus,* the genus *Halomonas,* the genus *Prevotella,* the genus *Dialister,* the genus *Anaerostipes,* the genus SMB53, the genus *Faecalibacterium,* the genus *Blautia,* the genus *Capnocytophaga,* the genus *Sphingomonas,* the genus *Lactobacillus,* the genus *Fimbriimonas,* the genus *Dermacoccus,* the genus *Achromobacter,* the genus *Novosphingobium,* the genus *Sneathia,* the genus *Agrobacterium,* the genus *Blastomonas,* the genus *Bdellovibrio,* the genus *Alkanindiges,* the genus *Roseateles,* and the genus *Shuttleworthia* was significantly different between colon cancer patients and normal individuals (see Example 4).

As a result of performing metagenomic analysis on bacteria-derived extracellular vesicles present in urine at a phylum level, the content of extracellular vesicles derived from bacteria belonging to the phylum *Proteobacteria* and the phylum *Euryarchaeota* was significantly different between colon cancer patients and normal individuals (see Example 5).

As a result of performing metagenomic analysis on bacteria-derived extracellular vesicles present in urine at a class level, the content of extracellular vesicles derived from bacteria belonging to the class *Gammaproteobacteria,* the class *Clostridia,* and the class *Methanobacteria* was significantly different between colon cancer patients and normal individuals (see Example 5).

As a result of performing metagenomic analysis on bacteria-derived extracellular vesicles present in urine at an order level, the content of extracellular vesicles derived from bacteria belonging to the order *Desulfobacterales,* the order *Stramenopiles,* the order *Pseudomonadales,* the order *Clostridiales,* the order *Turicibacterales,* the order *Desulfovibrionales,* the order *Oceanospirillales,* and the order *Methanobacteriales* was significantly different between colon cancer patients and normal individuals (see Example 5).

As a result of performing metagenomic analysis on bacteria-derived extracellular vesicles present in urine at a family level, the content of extracellular vesicles derived from bacteria belonging to the family *Moraxellaceae,* the family *Pseudomonadaceae,* the family *Streptococcaceae,* the family *Turicibacteraceae,* the family *Veillonellaceae,* the family *Bacteroidaceae,* the family *Aerococcaceae,* the family *Comamonadaceae,* the family *Clostridiaceae,* the family *Paraprevotellaceae,* the family *Christensenellaceae,* the family *Ruminococcaceae,* the family *Corynebacteriaceae,* the family *Gordoniaceae,* the family *Mycobacteriaceae,* the family *Desulfovibrionaceae,* the family *Halomonadaceae,* the family *Alcaligenaceae,* the family *Barnesiellaceae,* the family *Methanobacteriaceae,* and the family *Rikenellaceaewas* significantly different between colon cancer patients and normal individuals (see Example 5).

As a result of performing metagenomic analysis on bacteria-derived extracellular vesicles present in urine at a genus level, the content of extracellular vesicles derived from bacteria belonging to the genus *Rhizobium,* the genus *Proteus,* the genus *Morganella,* the genus *Acinetobacter,* the genus *Pseudomonas,* the genus SMB53, the genus *Enterococcus,* the genus *Lactococcus,* the genus *Turicibacter,* the genus *Coprococcus,* the genus *Bacteroides,* the genus *Dorea,* the genus *Streptococcus,* the genus *Lachnospira,* the genus *Ruminococcus,* the genus *Corynebacterium,* the genus *Comamonas,* the genus *Gordonia,* the genus *Paraprevotella,* the genus *Mycobacterium,* the genus *Roseburia,* the genus *Dialister,* the genus *Slackia,* the genus *Escherichia,* the genus *Phascolarctobacterium,* the genus *Sutterella,* the genus *Virgibacillus,* the genus *Eggerthella,* the genus *Halomonas,* the genus *Citrobacter,* the genus *Roseomonas,* the genus *Alloiococcus,* the genus *Serratia,* the genus *Methanobrevibacter,* and the genus *Bilophila* significantly different between colon cancer patients and normal individuals (see Example 5).

As a result of performing metagenomic analysis on bacteria-derived extracellular vesicles present in stool at a phylum level, the content of extracellular vesicles derived from bacteria belonging to the phylum *Spirochaetes* was significantly different between colon cancer patients and colon polyp patients (see Example 6).

As a result of performing metagenomic analysis on bacteria-derived extracellular vesicles present in stool at a class level, the content of extracellular vesicles derived from bacteria belonging to the class *Spirochaetes* and the class *Acidobacteria-6* was significantly different between colon cancer patients and colon polyp patients (see Example 6).

As a result of performing metagenomic analysis on bacteria-derived extracellular vesicles present in stool at an order level, the content of extracellular vesicles derived from bacteria belonging to the order *Spirochaetales* was significantly different between colon cancer patients and colon polyp patients (see Example 6).

As a result of performing metagenomic analysis on bacteria-derived extracellular vesicles present in stool at a family level, the content of extracellular vesicles derived from bacteria belonging to the family *Spirochaetaceae* and the family S24-7 was significantly different between colon cancer patients and colon polyp patients (see Example 6).

As a result of performing metagenomic analysis on bacteria-derived extracellular vesicles present in stool at a genus level, the content of extracellular vesicles derived from bacteria belonging to the genus *Treponema,* the genus *Dialister,* and the genus *Oscillospira* was significantly different between colon cancer patients and colon polyp patients (see Example 6).

As a result of performing metagenomic analysis on bacteria-derived extracellular vesicles present in urine at an order level, the content of extracellular vesicles derived from bacteria belonging to the order *Myxococcales* was significantly different between colon cancer patients and colon polyp patients (see Example 7).

As a result of performing metagenomic analysis on bacteria-derived extracellular vesicles present in urine at a genus level, the content of extracellular vesicles derived from bacteria belonging to the genus *Eubacterium* was significantly different between colon cancer patients and colon polyp patients (see Example 7).

As a result of performing metagenomic analysis on bacteria-derived extracellular vesicles present in stool at a phylum level, the content of extracellular vesicles derived from bacteria belonging to the phylum *Actinobacteria* was significantly different between colon polyp patients and normal individuals (see Example 8).

As a result of performing metagenomic analysis on bacteria-derived extracellular vesicles present in stool at a class level, the content of extracellular vesicles derived from bacteria belonging to the class *Betaproteobacteria* and the class *Solibacteres* was significantly different between colon polyp patients and normal individuals (see Example 8).

As a result of performing metagenomic analysis on bacteria-derived extracellular vesicles present in stool at an order level, the content of extracellular vesicles derived from bacteria belonging to the order *Burkholderiales,* the order *Sphingomonadales,* and the order *Solibacterales* was significantly different between colon polyp patients and normal individuals (see Example 8).

As a result of performing metagenomic analysis on bacteria-derived extracellular vesicles present in stool at a family level, the content of extracellular vesicles derived from bacteria belonging to the family *Rhizobiaceae* and the family *Sphingomonadaceae* was significantly different between colon polyp patients and normal individuals (see Example 8).

As a result of performing metagenomic analysis on bacteria-derived extracellular vesicles present in stool at a genus level, the content of extracellular vesicles derived from bacteria belonging to the genus *Sphingomonas,* the genus *Alkanindiges,* and the genus *Roseateles* was significantly different between colon polyp patients and normal individuals (see Example 8).

As a result of performing metagenomic analysis on bacteria-derived extracellular vesicles present in urine at a phylum level, the content of extracellular vesicles derived from bacteria belonging to the phylum *Proteobacteria* and the phylum *Euryarchaeota* was significantly different between colon polyp patients and normal individuals (see Example 9).

As a result of performing metagenomic analysis on bacteria-derived extracellular vesicles present in urine at a class level, the content of extracellular vesicles derived from bacteria belonging to the class *Gammaproteobacteria,* the class *Clostridia,* the class *Methanobacteria,* and the class 4C0d-2 was significantly different between colon polyp patients and normal individuals (see Example 9).

As a result of performing metagenomic analysis on bacteria-derived extracellular vesicles present in urine at an order level, the content of extracellular vesicles derived from bacteria belonging to the order *Stramenopiles,* the order *Pseudomonadales,* the order *Clostridiales,* the order *Oceanospirillales,* the order *Desulfovibrionales,* and the order *Methanobacteriales* was significantly different between colon polyp patients and normal individuals (see Example 9).

As a result of performing metagenomic analysis on bacteria-derived extracellular vesicles present in urine at a family level, the content of extracellular vesicles derived from bacteria belonging to the family Exiguobacteraceae, the family Moraxellaceae, the family Pseudomonadaceae, the family Rhizobiaceae, the family Streptococcaceae, the family Peptostreptococcaceae, the family Comamonadaceae, the family Veillonellaceae, the family Bacteroidaceae, the family Paraprevotellaceae, the family Ruminococcaceae, the family Corynebacteriaceae, the family Christensenellaceae, the family Odoribacteraceae, the family Desulfovibrionaceae, the family Halomonadaceae, the family Alcaligenaceae, the family Barnesiellaceae, the family Methanobacteriaceae, and the family Rikenellaceae was significantly different between colon polyp patients and normal individuals (see Example 9).

As a result of performing metagenomic analysis on bacteria-derived extracellular vesicles present in urine at a genus level, the content of extracellular vesicles derived from bacteria belonging to the genus *Rhizobium,* the genus *Morganella,* the genus *Proteus,* the genus *Exiguobacterium,* the genus *Acinetobacter,* the genus *Pseudomonas,* the genus SMB53, the genus *Lactococcus,* the genus *Coprococcus,* the genus *Streptococcus,* the genus *Bacteroides,* the genus *Ruminococcus,* the genus *Corynebacterium,* the genus *Odoribacter,* the genus *Clostridium,* the genus *Comamonas,* the genus *Paraprevotella,* the genus *Roseburia,* the genus *Citrobacter,* the genus *Klebsiella,* the genus *Virgibacillus,* the genus *Slackia,* the genus *Dialister,* the genus *Phascolarctobacterium,* the genus *Sutterella,* the genus *Halomonas,* the genus *Roseomonas,* and the genus *Methanobrevibacter* was significantly different between colon polyp patients and normal individuals (see Example 9).

From the above-described example results, it was confirmed that bacteria-derived extracellular vesicles exhibiting a significant change in content in colon cancer patients compared to normal individuals and colon polyp patients were identified by performing metagenomic analysis on bacteria-derived extracellular vesicles isolated from stool and urine, and colon cancer could be diagnosed by analyzing an increase or decrease in the content of bacteria-derived extracellular vesicles at each level through metagenomic analysis.

From the above-described example results, it was also confirmed that bacteria-derived extracellular vesicles exhibiting a significant change in content in colon polyp patients compared to normal individuals were identified by performing metagenomic analysis on bacteria-derived extracellular vesicles isolated from stool and urine, and a colon polyp could be diagnosed by analyzing an increase or decrease in the content of bacteria-derived extracellular vesicles at each level through metagenomic analysis.

Hereinafter, the present invention will be described with reference to exemplary examples to aid in understanding of the present invention. However, these examples are provided only for illustrative purposes and are not intended to limit the scope of the present invention.

### [Examples]

### Example 1. Analysis of In Vivo Absorption, Distribution, and Excretion Patterns of Intestinal Bacteria and Bacteria-Derived Extracellular Vesicles

To evaluate whether intestinal bacteria and bacteria-derived extracellular vesicles are systematically absorbed through the gastrointestinal tract, an experiment was conducted using the following method. More particularly, 50 µg of each of intestinal bacteria and the bacteria-derived extracellular vesicles (EVs), labeled with fluorescence, were orally administered to the gastrointestinal tracts of mice, and fluorescence was measured at 0 h, and after 5 min, 3 h, 6 h, and 12 h. As a result of observing the entire images of mice, as illustrated in FIG. 1A, the bacteria were not systematically absorbed when administered, while the bacteria-derived EVs were systematically absorbed at 5 min after administration, and, at 3 h after administration, fluorescence was strongly observed in the bladder, from which it was confirmed that the EVs were excreted via the urinary system, and were present in the bodies up to 12 h after administration.

After intestinal bacteria and intestinal bacteria-derived extracellular vesicles were systematically absorbed, to evaluate a pattern of invasion of intestinal bacteria and the bacteria-derived EVs into various organs in the human body after being systematically absorbed, 50 µg of each of the bacteria and bacteria-derived EVs, labeled with fluorescence, were administered using the same method as that used above, and then, at 12 h after administration, blood, the heart, the lungs, the liver, the kidneys, the spleen, adipose tissue, and muscle were extracted from each mouse. As a result of observing fluorescence in the extracted tissues, as illustrated in FIG. 1B, it was confirmed that the intestinal bacteria were not absorbed into each organ, while the bacteria-derived EVs were distributed in the blood, heart, lungs, liver, kidneys, spleen, adipose tissue, and muscle.

### Example 2. Vesicle Isolation and DNA Extraction from Stool and Urine

To isolate extracellular vesicles and extract DNA, from stool and urine, first, stool and urine was added to a 10 ml tube and centrifuged at 3,500 x g and 4 °C for 10 min to precipitate a suspension, and only a supernatant was collected, which was then placed in a new 10 ml tube. The collected supernatant was filtered using a 0.22 µm filter to remove bacteria and impurities, and then placed in centripreigugal filters (50 kD) and centrifuged at 1500 x g and 4 °C for 15 min to discard materials with a smaller size than 50 kD, and then concentrated to 10 ml. Once again, bacteria and impurities were removed therefrom using a 0.22 µm filter, and then the resulting concentrate was subjected to ultra-high speed centrifugation at 150,000 x g and 4 °C for 3 hours by using a Type 90ti rotor to remove a supernatant, and the agglomerated pellet was dissolved with phosphate-buffered saline (PBS), thereby obtaining vesicles.

100 µl of the extracellular vesicles isolated from the stool and urine according to the above-described method was boiled at 100 °C to allow the internal DNA to come out of the lipid and then cooled on ice. Next, the resulting vesicles were centrifuged at 10,000 x g and 4 °C for 30 minutes to remove the remaining suspension, only the supernatant was collected, and then the amount of DNA extracted was quantified using a NanoDrop sprectrophotometer. In addition, to verify whether bacteria-derived DNA was present in the extracted DNA, PCR was performed using 16s rDNA primers shown in Table 1 below.

**[Table 1]**

| Primer | | Sequence | SEQ ID NO. |
|---|---|---|---|
| 16S rDNA | 16S_V3_F | 5'-TCGTCGGCAGCGTC AGATGTGTATAAGAG ACAGCCTACGGGNGG CWGCAG-3' | 1 |
| | 16S_V4_R | 5'-GTCTCGTGGGCTCG GAGATGTGTATAAGA GACAGGACTACHVGG GTATCTAATCC-3' | 2 |

### Example 3. Metagenomic Analysis Using DNA Extracted from Vesicle in Stool and Urine

DNA was extracted using the same method as that used in Example 2, and then PCR was performed thereon using 16S rDNA primers shown in Table 1 to amplify DNA, followed by sequencing (Illumina MiSeq sequencer). The results were output as standard flowgram format (SFF) files, and the SFF files were converted into sequence files (.fasta) and nucleotide quality score files using GS FLX software (v2.9), and then credit rating for reads was identified, and portions with a window (20 bps) average base call accuracy of less than 99% (Phred score <20) were removed. After removing the low-quality portions, only reads having a length of 300 bps or more were used (Sickle version 1.33), and, for operational taxonomy unit (OTU) analysis, clustering was performed using UCLUST and USEARCH according to sequence similarity. In particular, clustering was performed based on sequence similarity values of 94% for genus, 90% for family, 85% for order, 80% for class, and 75% for phylum, and phylum, class, order, family, and genus levels of each OTU were classified, and bacteria with a sequence similarity of 97% or more were analyzed (QIIME) using 16S DNA sequence databases (108,453 sequences) of BLASTN and GreenGenes.

### Example 4. Colon Cancer Diagnostic Model Based on Metagenomic Analysis of Bacteria-Derived EVs Isolated from Stool of Normal Individuals and Colon Cancer Patients

EVs were isolated from stool samples of 29 colon cancer patients and 358 normal individuals, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

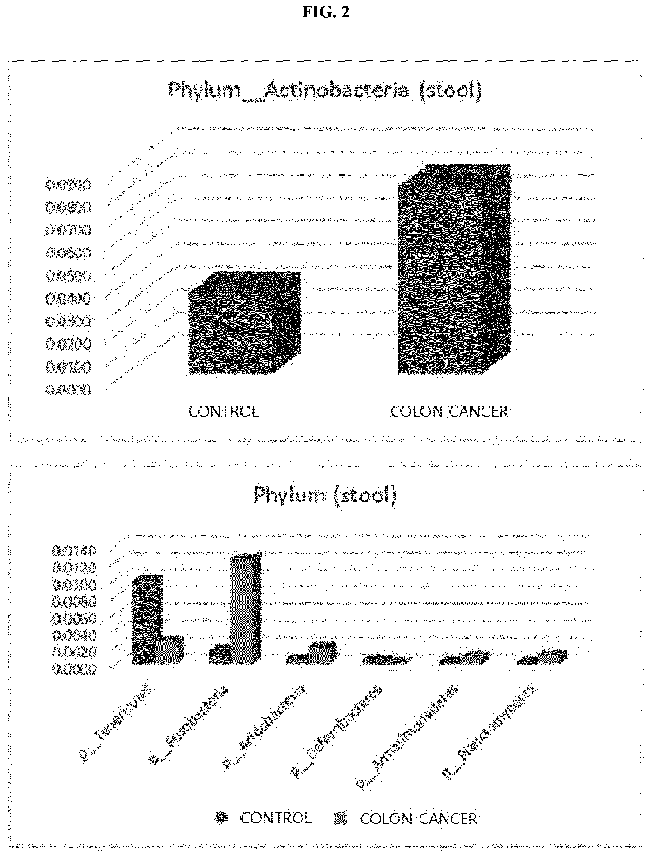

As a result of analyzing bacteria-derived extracellular vesicles in stool at a phylum level, a diagnostic model developed using, as a biomarker, one or more bacteria from the phylum *Deferribacteres,* the phylum *Tenericutes,* the phylum *Actinobacteria,* the phylum *Acidobacteria,* the phylum *Armatimonadetes,* the phylum *Planctomycetes,* and the phylum *Fusobacteria* exhibited significant diagnostic performance for colon cancer (see Table 2 and FIG. 2).

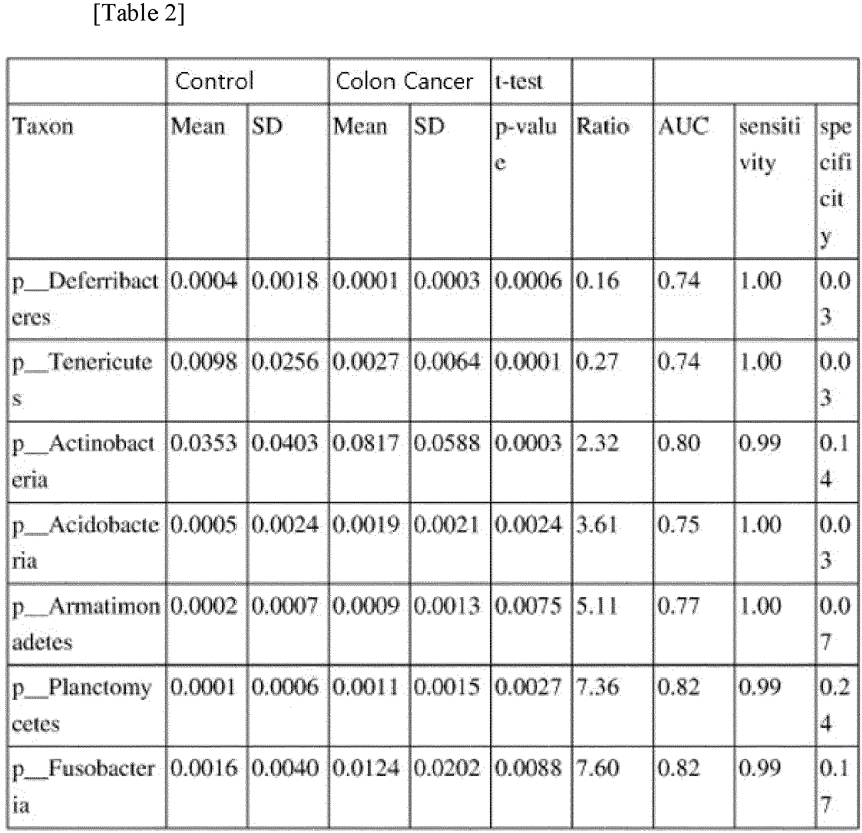

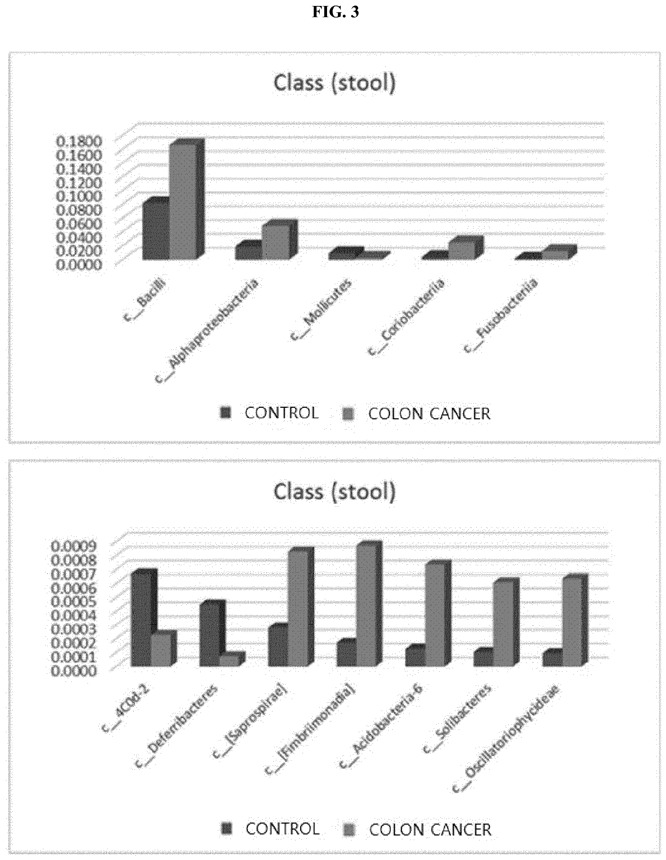

As a result of analyzing bacteria-derived extracellular vesicles in stool at a class level, a diagnostic model developed using, as a biomarker, one or more bacteria from the class *Deferribacteres,* the class *Mollicutes,* the class 4C0d-2, the class *Bacilli,* the class *Alphaproteobacteria,* the class *Saprospirae,* the class *Fimbriimonadia,* the class *Acidobacteria-6,* the class *Solibacteres,* the class *Coriobacteriia,* the class *Oscillatoriophycideae,* and the class *Fusobacteriia* exhibited significant diagnostic performance for colon cancer (see Table 3 and FIG. 3).

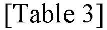

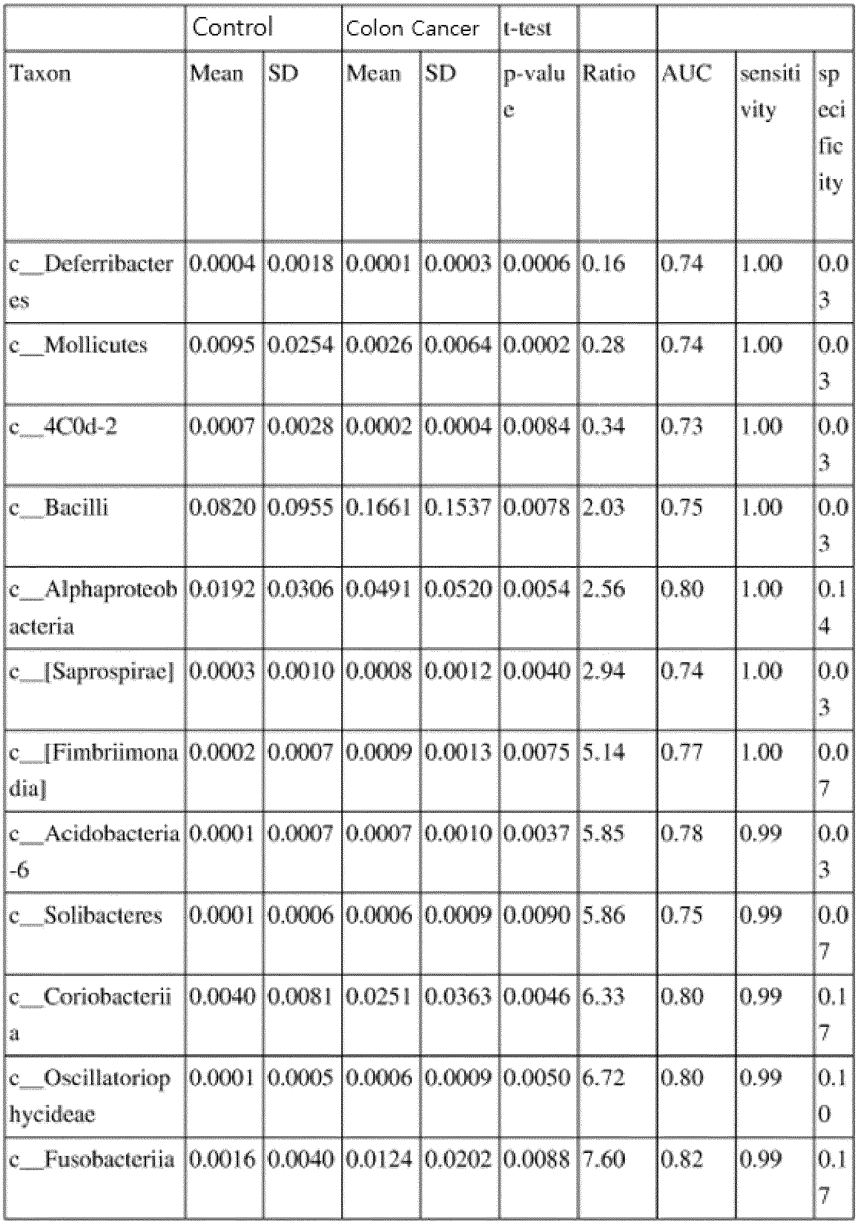

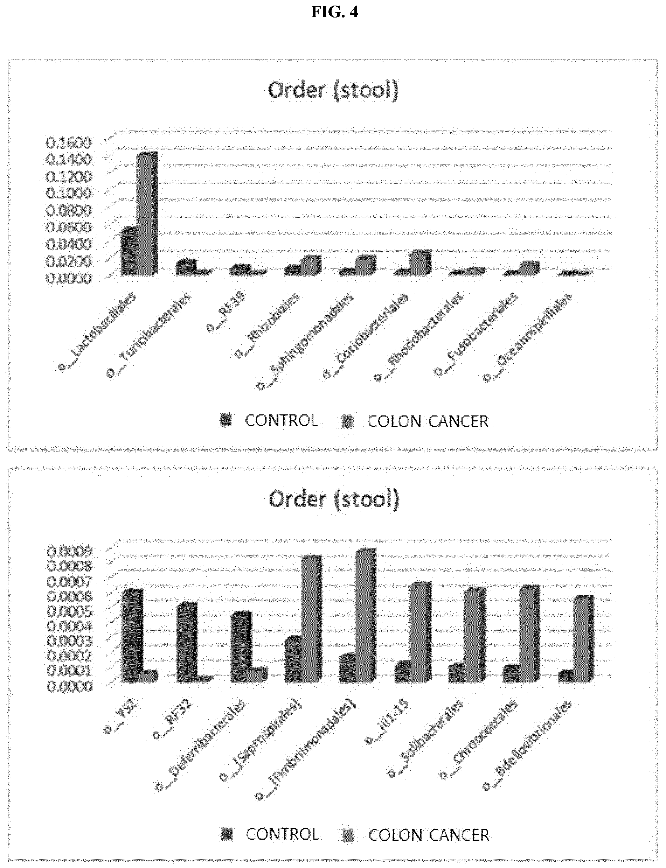

As a result of analyzing bacteria-derived extracellular vesicles in stool at an order level, a diagnostic model developed using, as a biomarker, one or more bacteria from the order RF32, the order YS2, the order *Deferribacterales,* the order *Turicibacterales,* the order RF39, the order *Oceanospirillales,* the order *Rhizobiales,* the order *Lactobacillales,* the order *Rhodobacterales,* the order *Saprospirales,* the order *Sphingomonadales,* the order *Fimbriimonadales,* the order iii1-15, the order *Solibacterales,* the order *Coriobacteriales,* the order *Chroococcales,* the order *Fusobacteriales,* and the order *Bdellovibrionales* exhibited significant diagnostic performance for colon cancer (see Table 4 and FIG. 4).

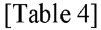

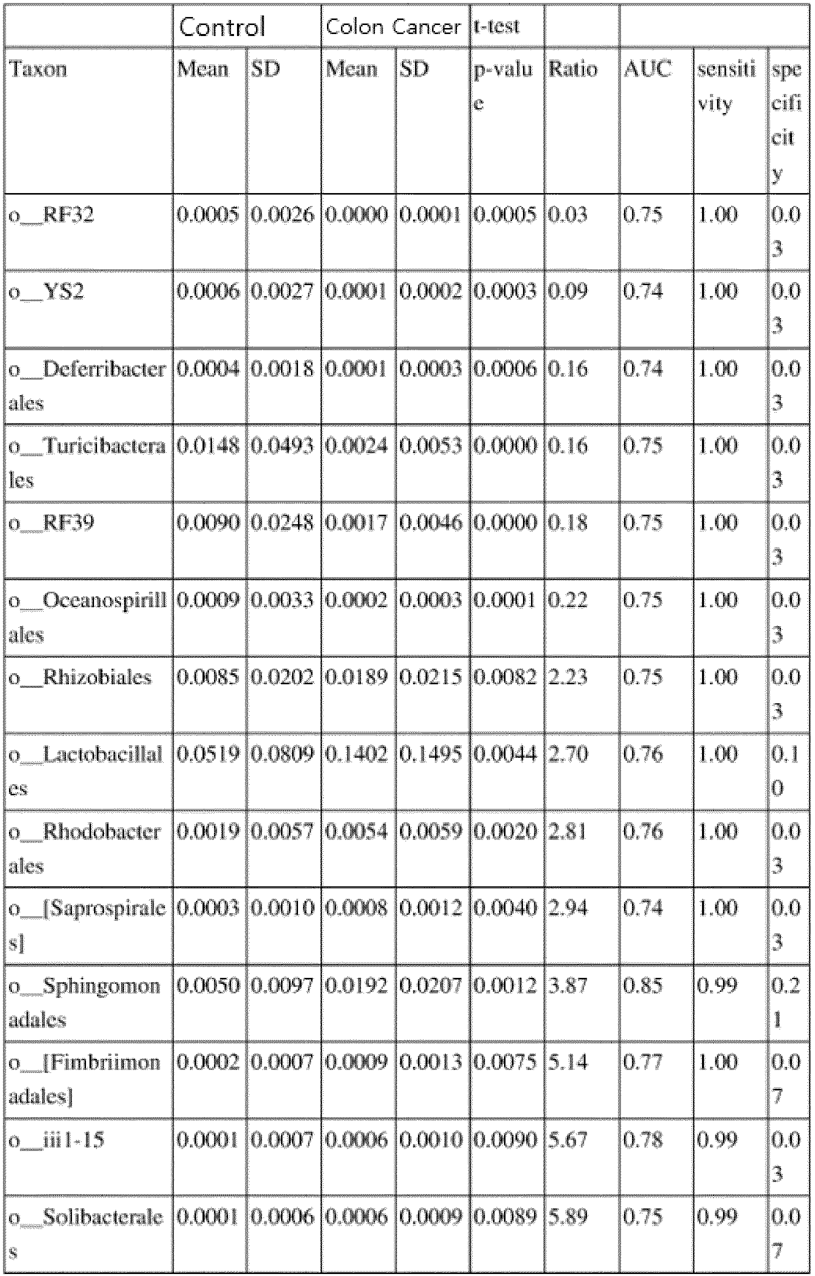

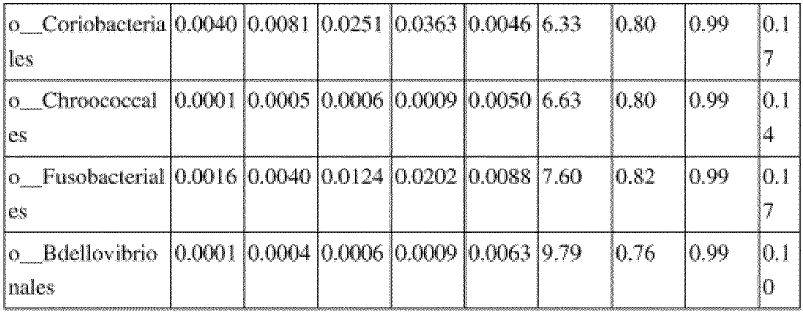

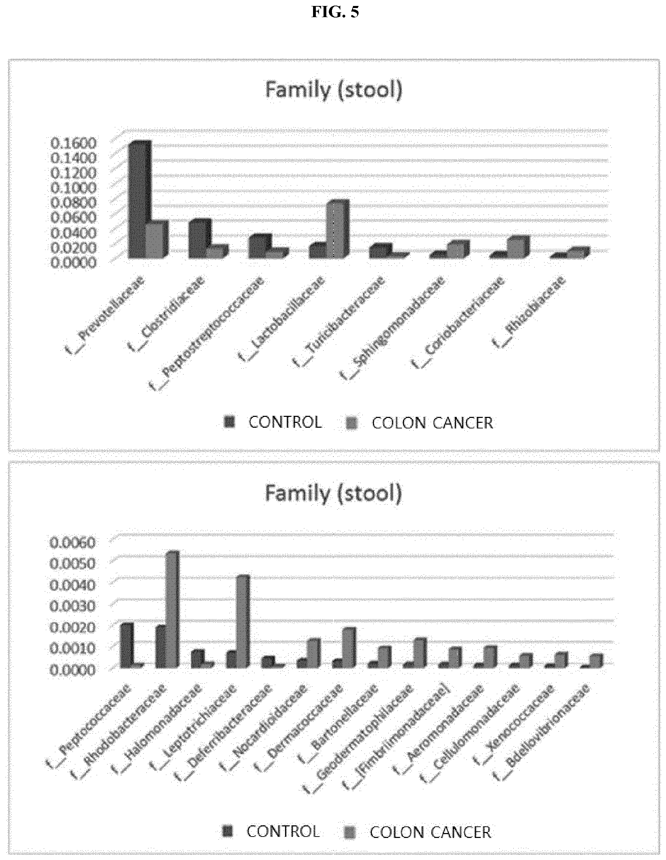

As a result of analyzing bacteria-derived extracellular vesicles in stool at a family level, a diagnostic model developed using, as a biomarker, one or more bacteria from the family *Peptococcaceae,* the family *Deferribacteraceae,* the family *Turicibacteraceae,* the family *Halomonadaceae,* the family *Clostridiaceae,* the family *Prevotellaceae,* the family *Peptostreptococcaceae,* the family *Rhodobacteraceae,* the family *Nocardioidaceae,* the family *Sphingomonadaceae,* the family *Bartonellaceae,* the family *Cellulomonadaceae,* the family *Lactobacillaceae,* the family *Rhizobiaceae,* the family *Fimbriimonadaceae,* the family *Dermacoccaceae,* the family *Leptotrichiaceae,* the family *Coriobacteriaceae,* the family *Xenococcaceae,* the family *Aeromonadaceae,* the family *Geodermatophilaceae,* and the family *Bdellovibrionaceae* exhibited significant diagnostic performance for colon cancer (see Table 5 and FIG. 5).

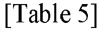

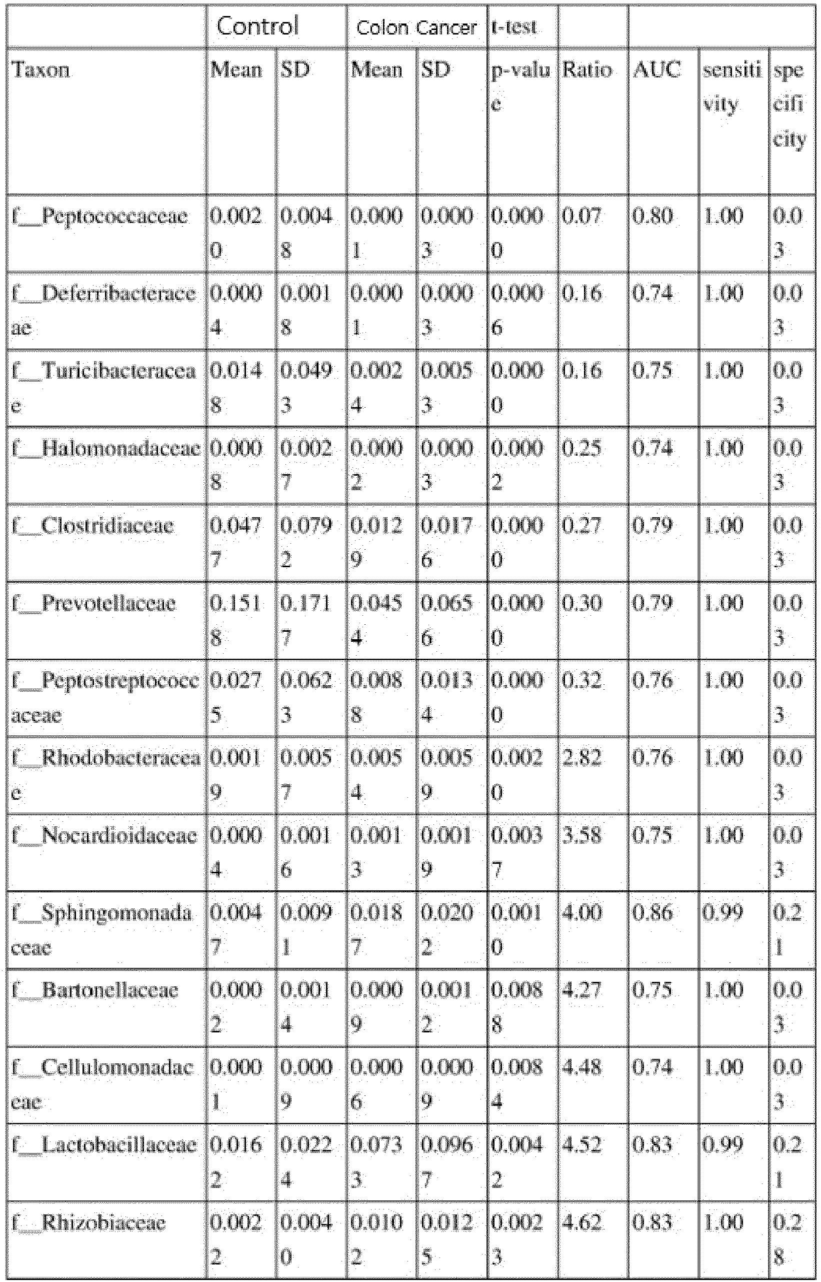

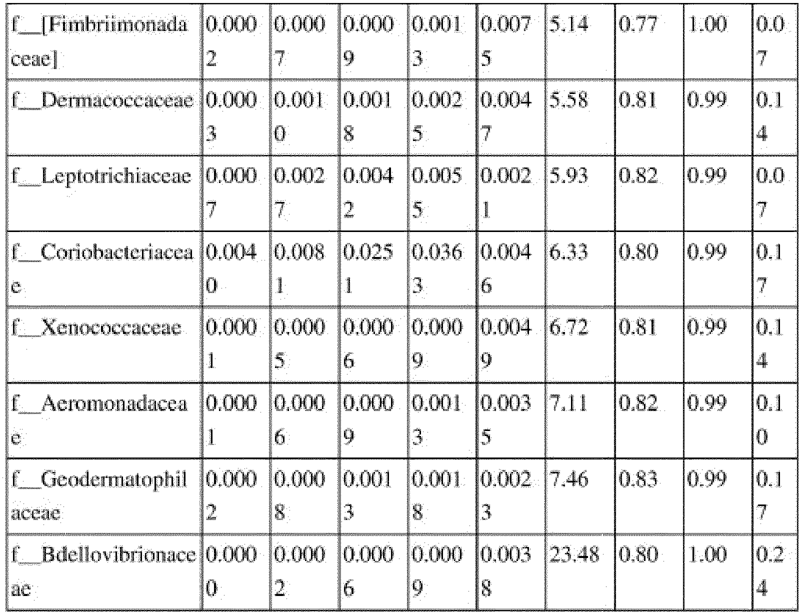

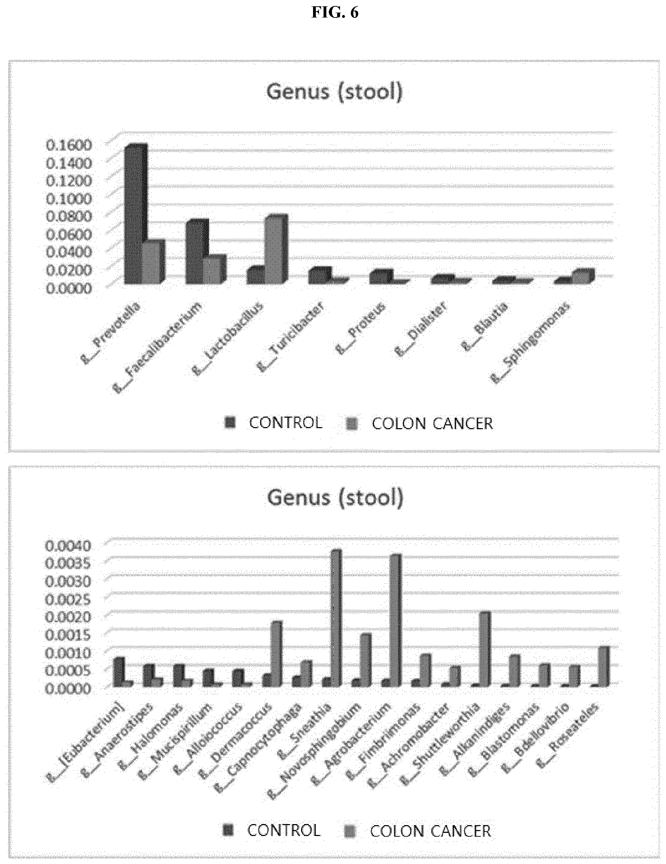

As a result of analyzing bacteria-derived extracellular vesicles in stool at a genus level, a diagnostic model developed using, as a biomarker, one or more bacteria from the genus rc4-4, the genus *Proteus,* the genus *Catenibacterium,* the genus *Mucispirillum,* the genus *Eubacterium,* the genus *Turicibacter,* the genus *Alloiococcus,* the genus *Halomonas,* the genus *Prevotella,* the genus *Dialister,* the genus *Anaerostipes,* the genus SMB53, the genus *Faecalibacterium,* the genus *Blautia,* the genus *Capnocytophaga,* the genus *Sphingomonas,* the genus *Lactobacillus,* the genus *Fimbriimonas,* the genus *Dermacoccus,* the genus *Achromobacter,* the genus *Novosphingobium,* the genus *Sneathia,* the genus *Agrobacterium,* the genus *Blastomonas,* the genus *Bdellovibrio,* the genus *Alkanindiges,* the genus *Roseateles,* and the genus *Shuttleworthia* exhibited significant diagnostic performance for colon cancer (see Table 6 and FIG. 6).

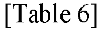

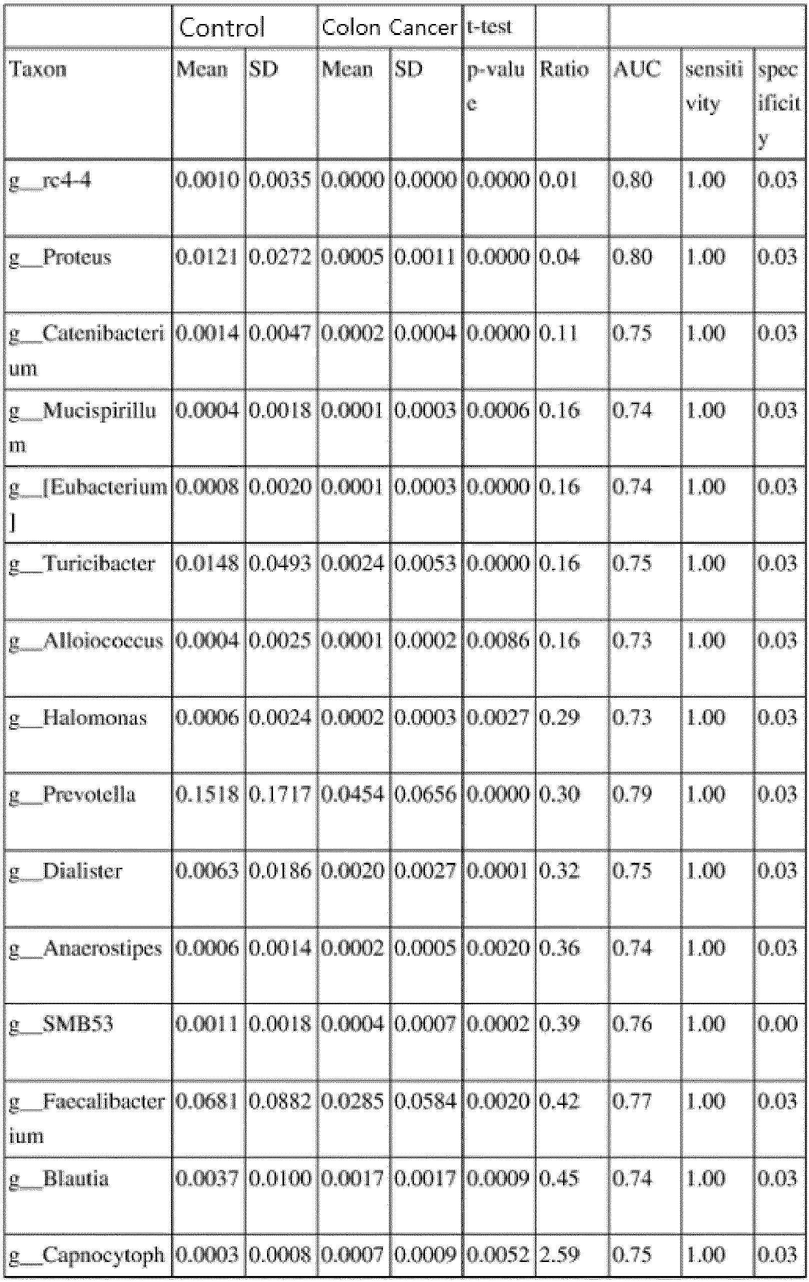

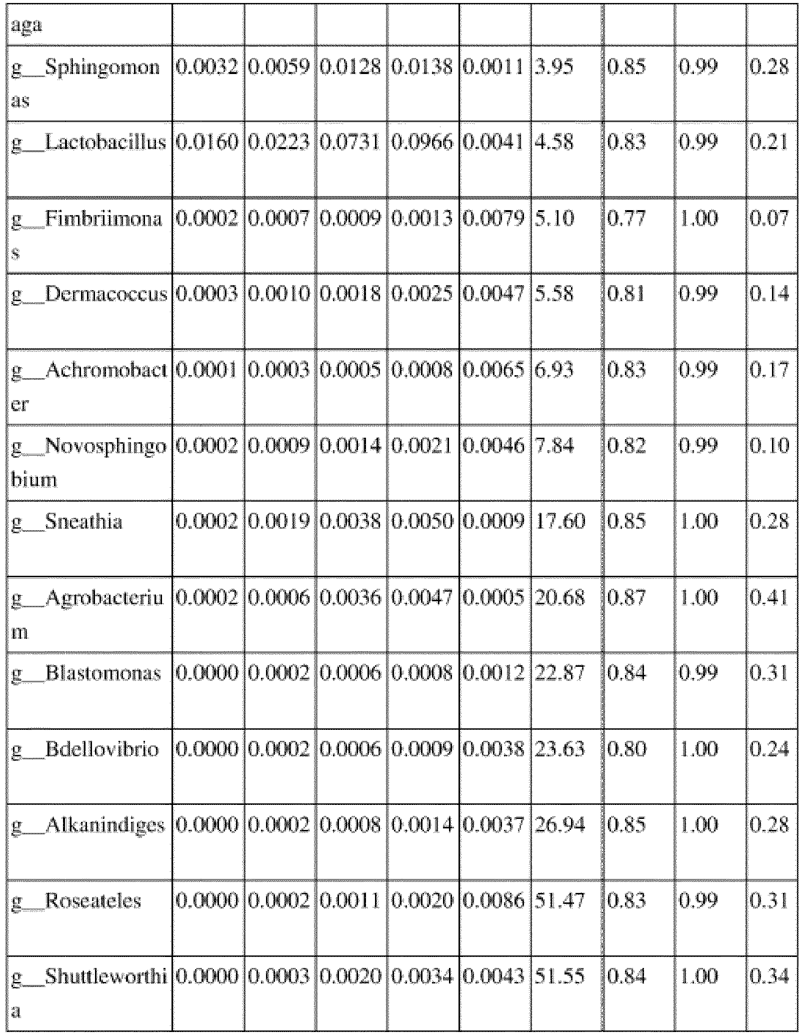

### Example 5. Colon Cancer Diagnostic Model Based on Metagenomic Analysis of Bacteria-Derived EVs Isolated from Urine of Normal Individuals and Colon Cancer Patients

Extracellular vesicles were isolated from urine samples of 38 colon cancer patients and 38 normal individuals, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

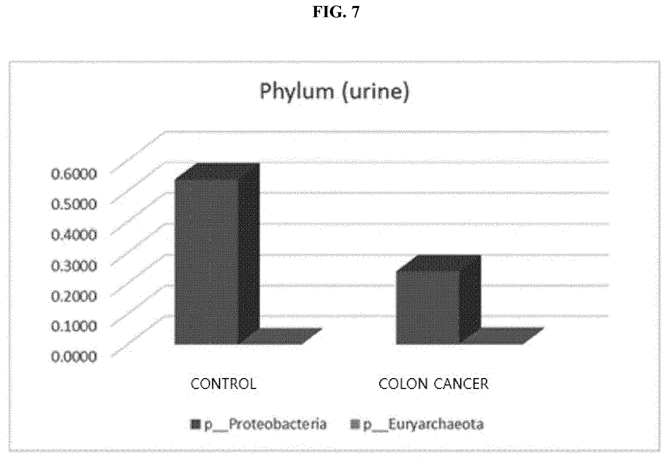

As a result of analyzing bacteria-derived extracellular vesicles in urine at a phylum level, a diagnostic model developed using, as a biomarker, one or more bacteria from the phylum *Proteobacteria* and the phylum *Euryarchaeota* exhibited significant diagnostic performance for colon cancer (see Table 7 and FIG. 7).

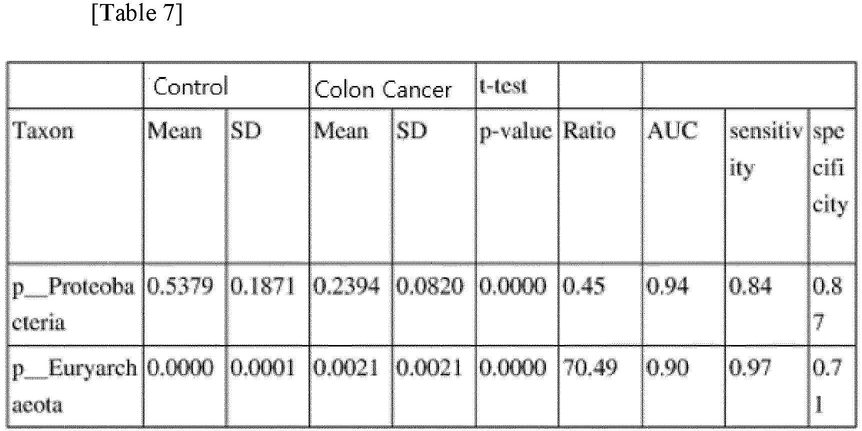

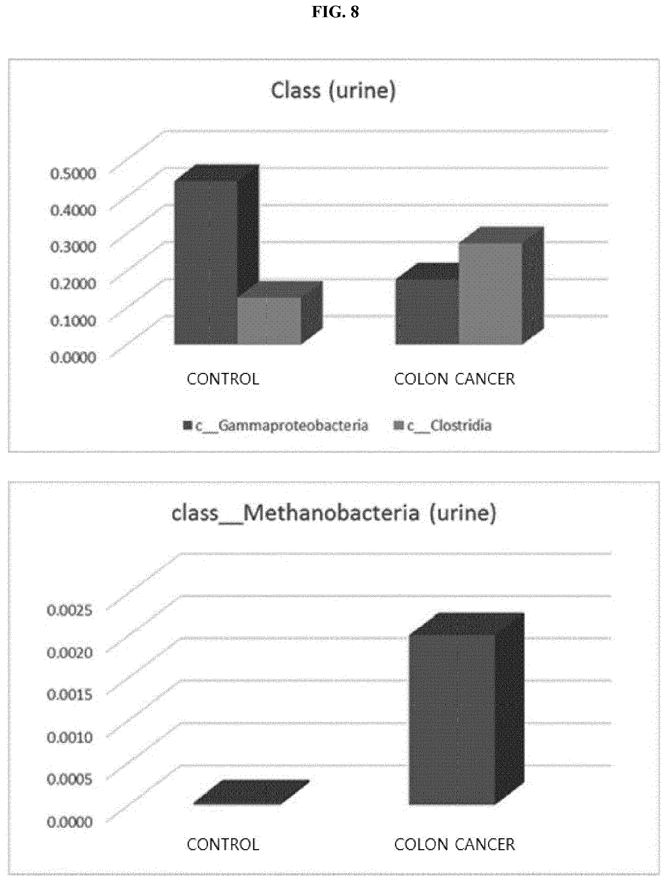

As a result of analyzing bacteria-derived extracellular vesicles in urine at a class level, a diagnostic model developed using, as a biomarker, one or more bacteria from the class *Gammaproteobacteria,* the class *Clostridia,* and the class *Methanobacteria* exhibited significant diagnostic performance for colon cancer (see Table 8 and FIG. 8).

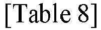

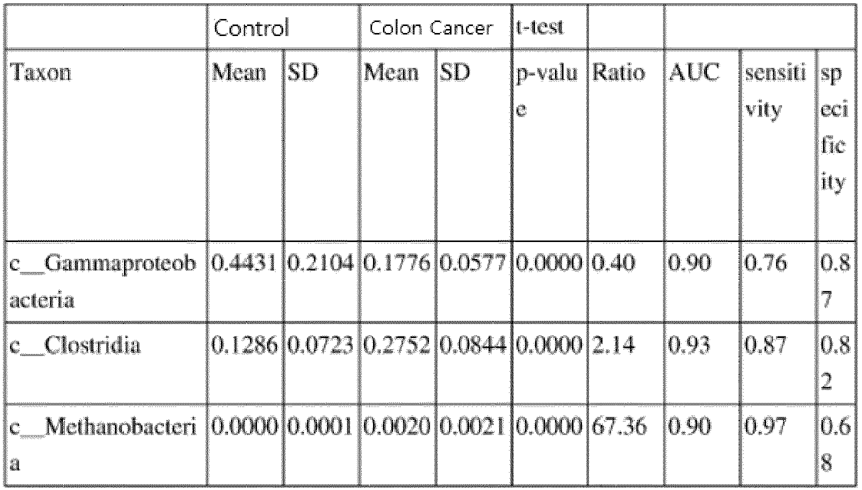

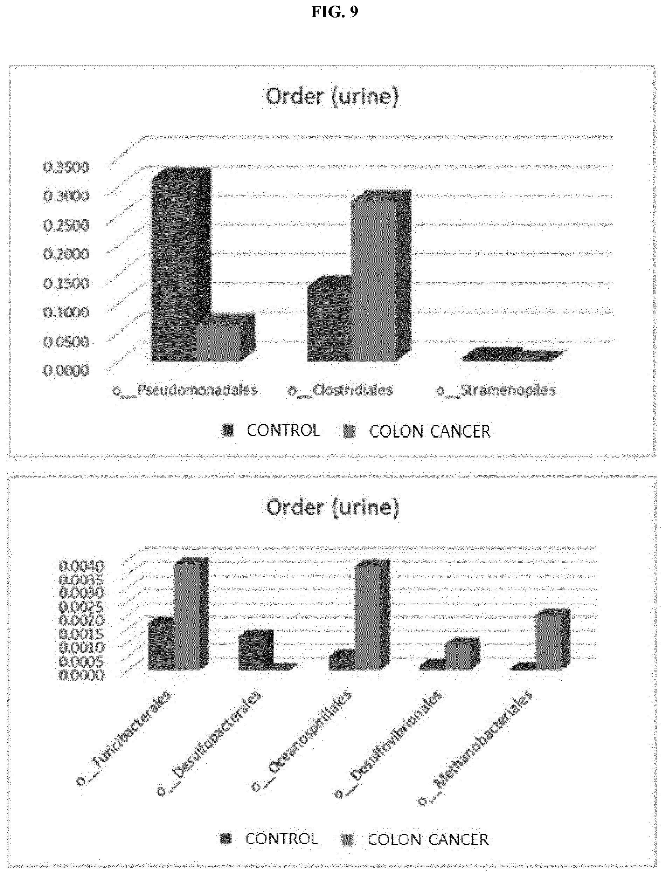

As a result of analyzing bacteria-derived extracellular vesicles in urine at an order level, a diagnostic model developed using, as a biomarker, one or more bacteria from the class *Desulfobacterales,* the class *Stramenopiles,* the class *Pseudomonadales,* the class *Clostridiales,* the class *Turicibacterales,* the class *Desulfovibrionales,* the class *Oceanospirillales,* and the class *Methanobacteriales* exhibited significant diagnostic performance for colon cancer (see Table 9 and FIG. 9).

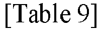

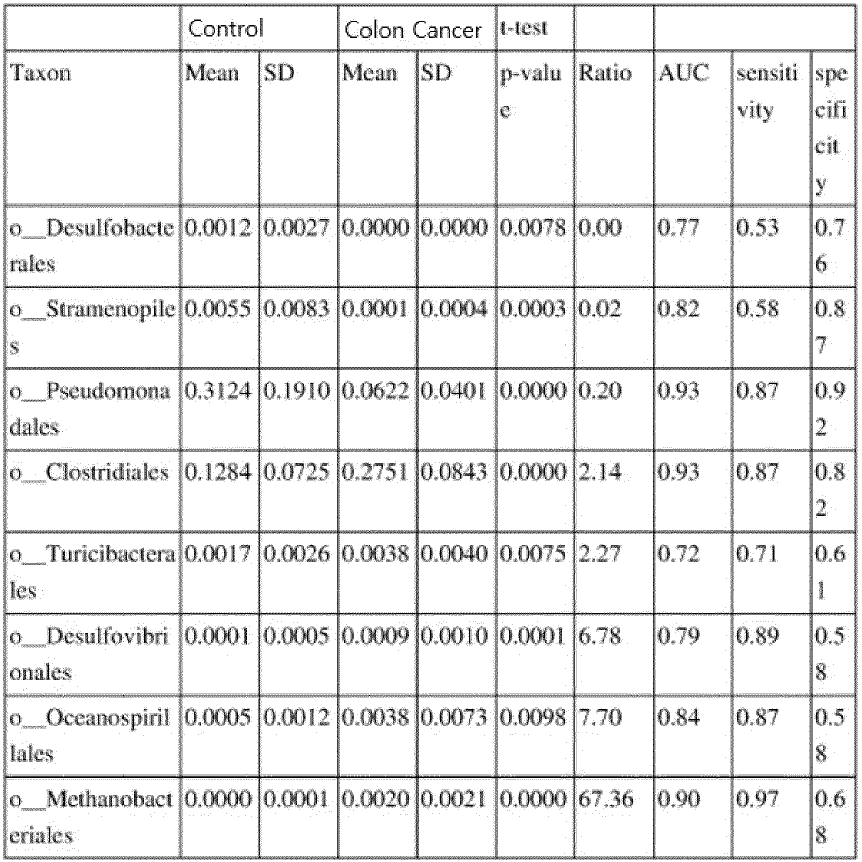

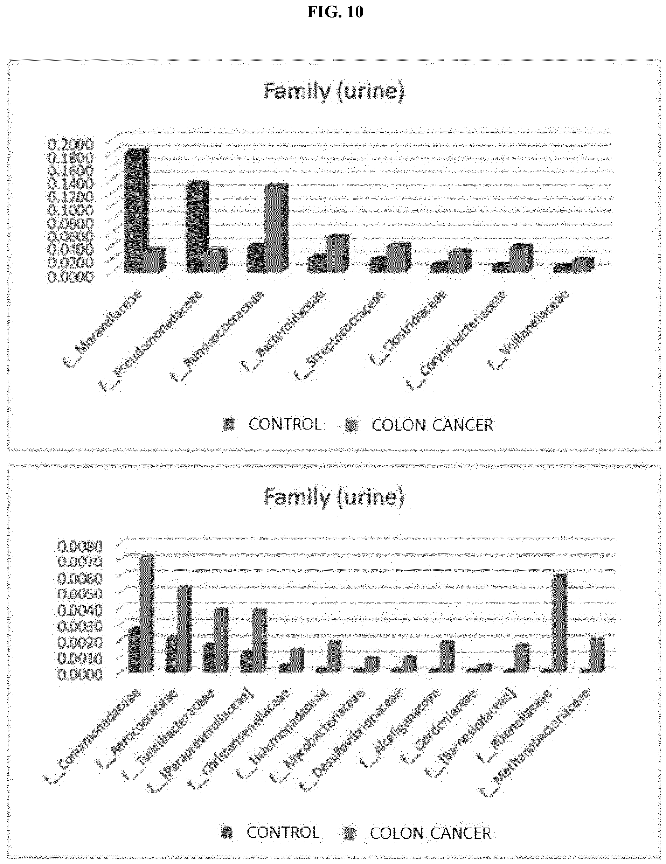

As a result of analyzing bacteria-derived extracellular vesicles in urine at a family level, a diagnostic model developed using, as a biomarker, one or more bacteria from the family *Moraxellaceae,* the family *Pseudomonadaceae,* the family *Streptococcaceae,* the family *Turicibacteraceae,* the family *Veillonellaceae,* the family *Bacteroidaceae,* the family *Aerococcaceae,* the family *Comamonadaceae,* the family *Clostridiaceae,* the family *Paraprevotellaceae,* the family *Christensenellaceae,* the family *Ruminococcaceae,* the family *Corynebacteriaceae,* the family *Gordoniaceae,* the family *Mycobacteriaceae,* the family *Desulfovibrionaceae,* the family *Halomonadaceae,* the family *Alcaligenaceae,* the family *Barnesiellaceae,* the family *Methanobacteriaceae,* and the family *Rikenellaceaeexhibited* significant diagnostic performance for colon cancer (see Table 10 and FIG. 10).

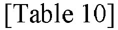

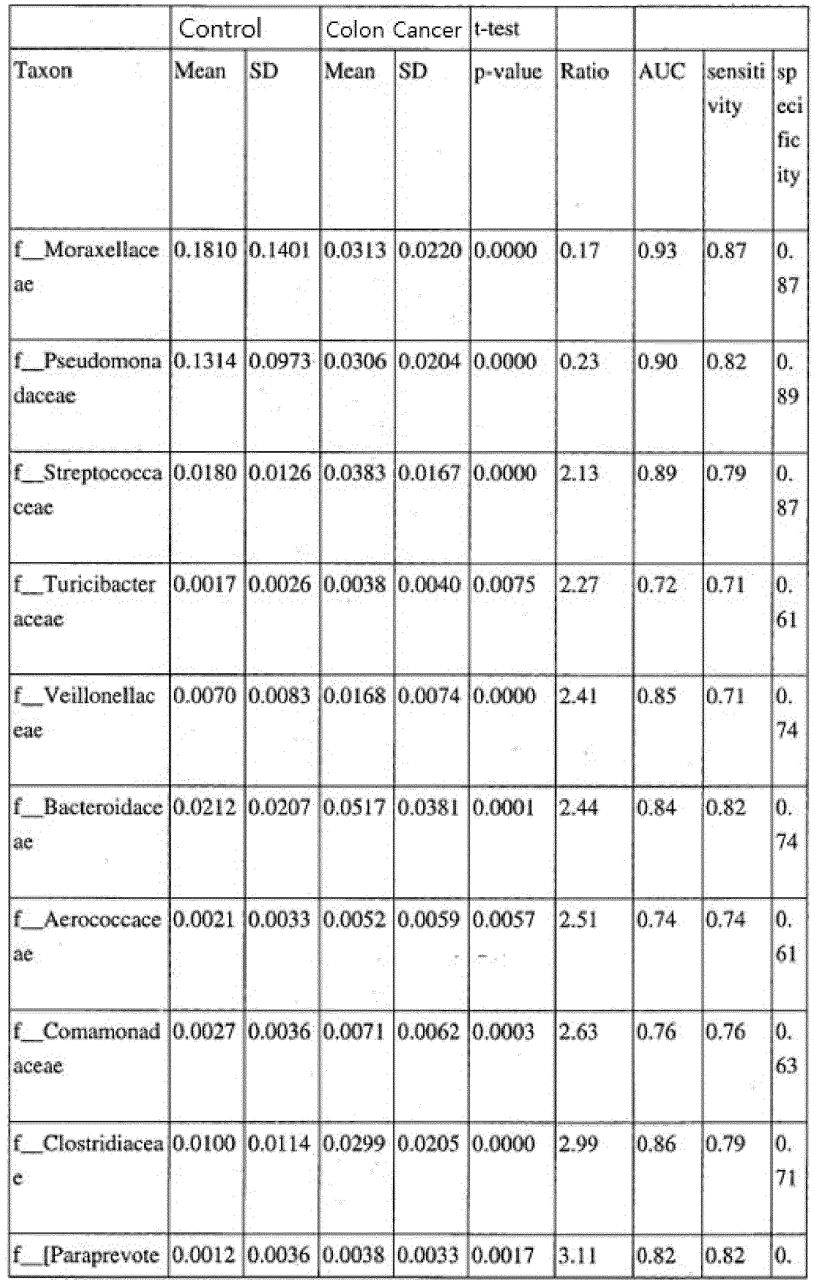

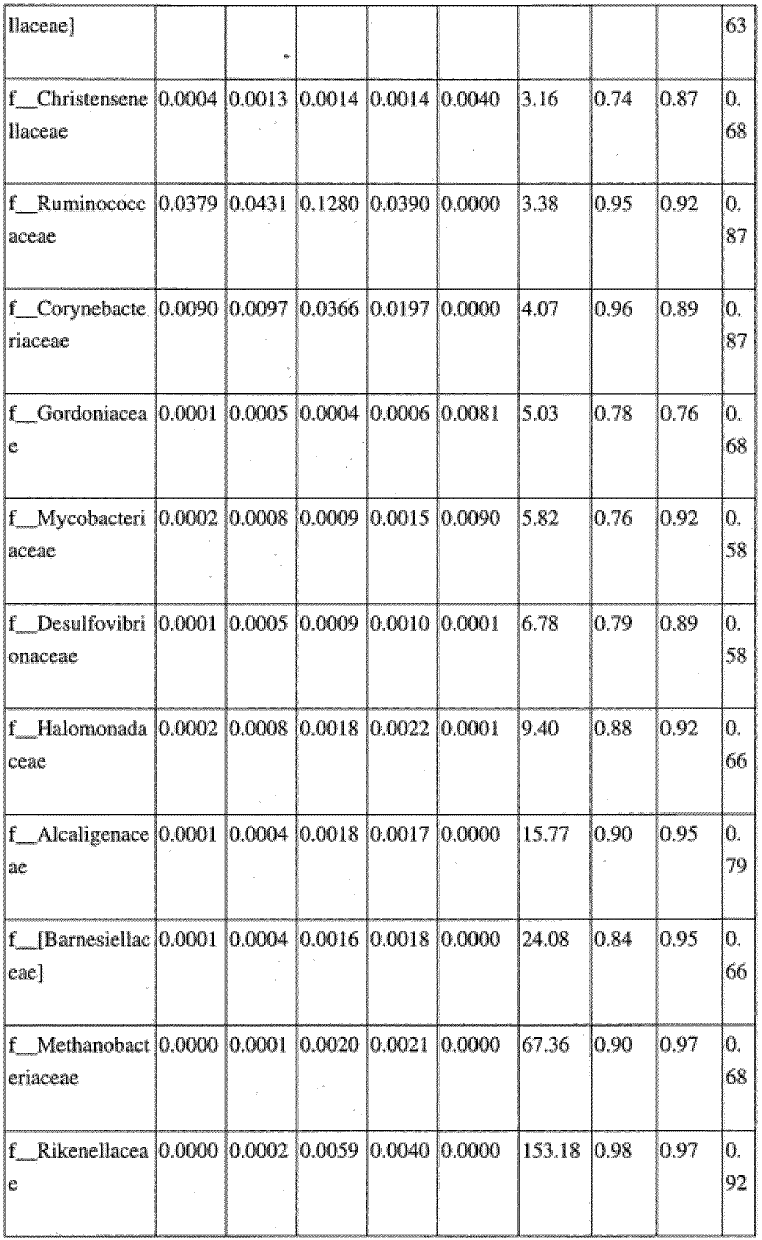

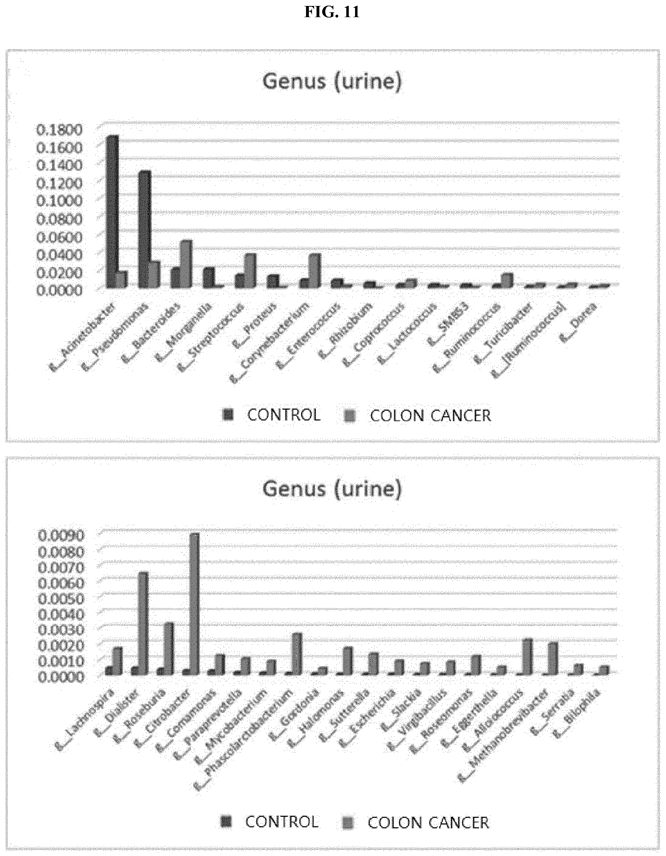

As a result of analyzing bacteria-derived extracellular vesicles in urine at a genus level, a diagnostic model developed using, as a biomarker, one or more bacteria from the genus *Rhizobium,* the genus *Proteus,* the genus *Morganella,* the genus *Acinetobacter,* the genus *Pseudomonas,* the genus SMB53, the genus *Enterococcus,* the genus *Lactococcus,* the genus *Turicibacter,* the genus *Coprococcus,* the genus *Bacteroides,* the genus *Dorea,* the genus *Streptococcus,* the genus *Lachnospira,* the genus *Ruminococcus,* the genus *Corynebacterium,* the genus *Comamonas,* the genus *Gordonia,* the genus *Paraprevotella,* the genus *Mycobacterium,* the genus *Roseburia,* the genus *Dialister,* the genus *Slackia,* the genus *Escherichia,* the genus *Phascolarctobacterium,* the genus *Sutterella,* the genus *Virgibacillus,* the genus *Eggerthella,* the genus *Halomonas,* the genus *Citrobacter,* the genus *Roseomonas,* the genus *Alloiococcus,* the genus *Serratia,* the genus *Methanobrevibacter,* and the genus *Bilophila* significant diagnostic performance for colon cancer (see Table 11 and FIG. 11).

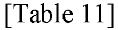

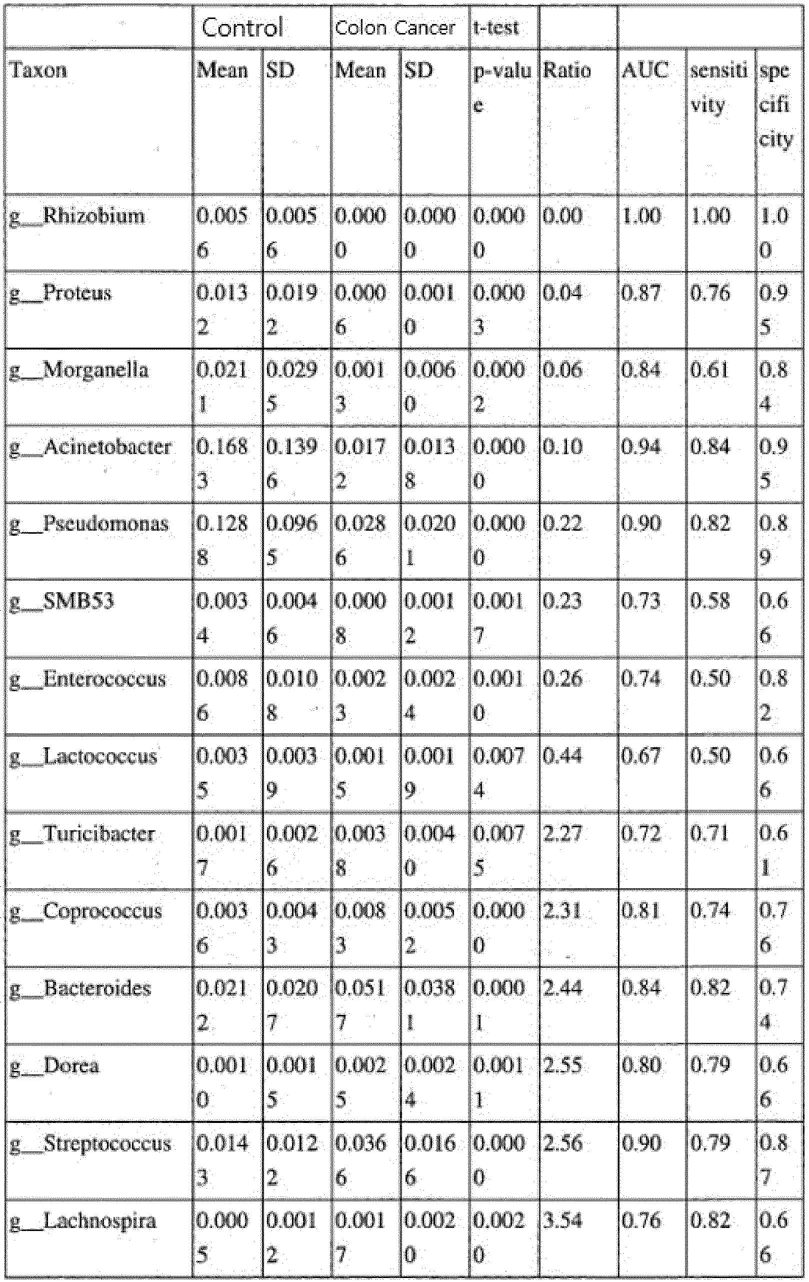

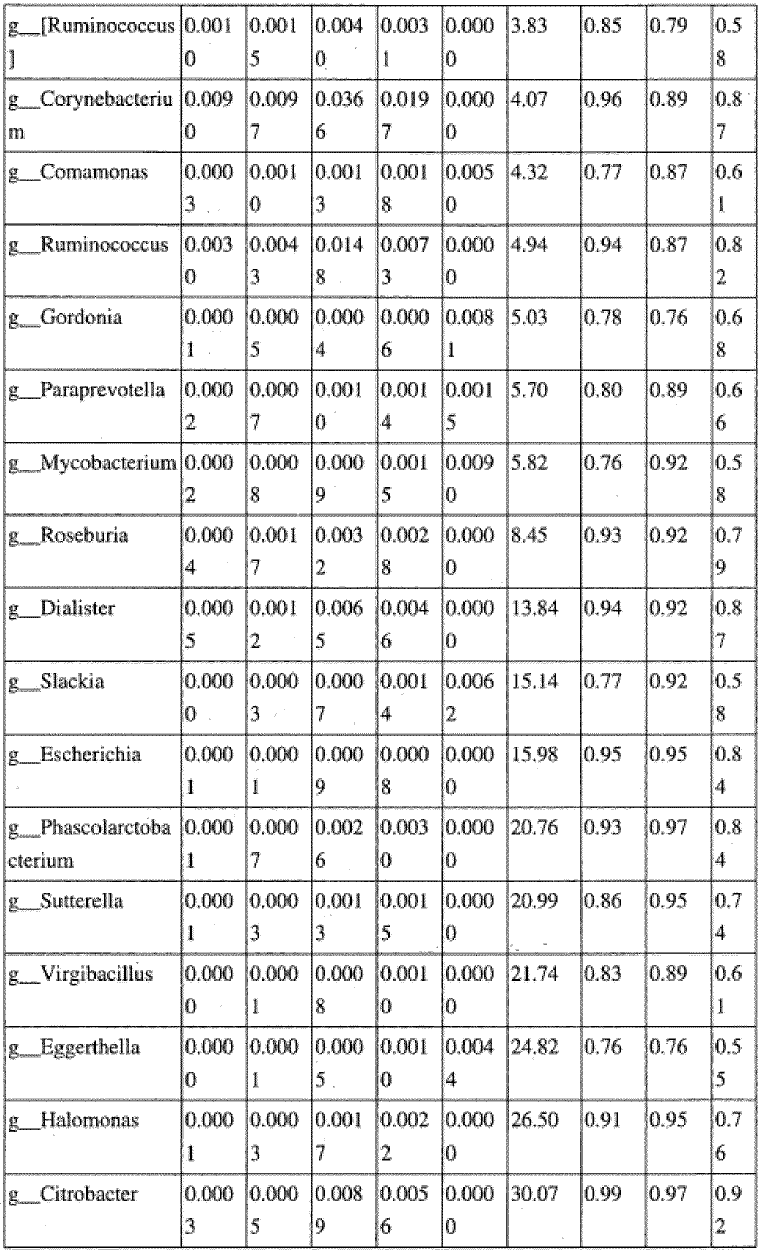

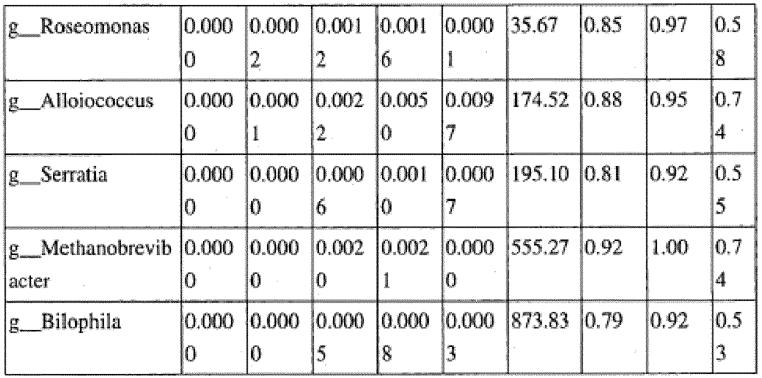

### Example 6. Colon Cancer Diagnostic Model Based on Metagenomic Analysis of Bacteria-Derived EVs Isolated from Stool of Colon Polyp Patients and Colon Cancer Patients

Extracellular vesicles were isolated from stool samples of 29 colon cancer patients and 27 colon polyp patients, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

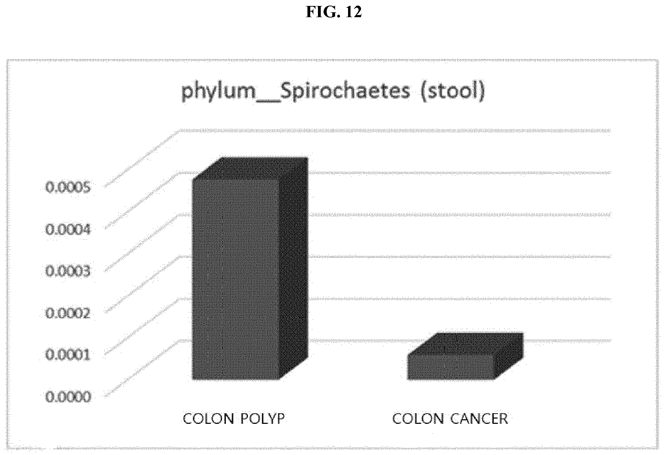

As a result of analyzing bacteria-derived extracellular vesicles in stool at a phylum level, a diagnostic model developed using bacteria belonging to the phylum *Spirochaetes* as a biomarker exhibited significant diagnostic performance for colon cancer (see Table 12 and FIG. 12).

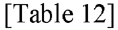

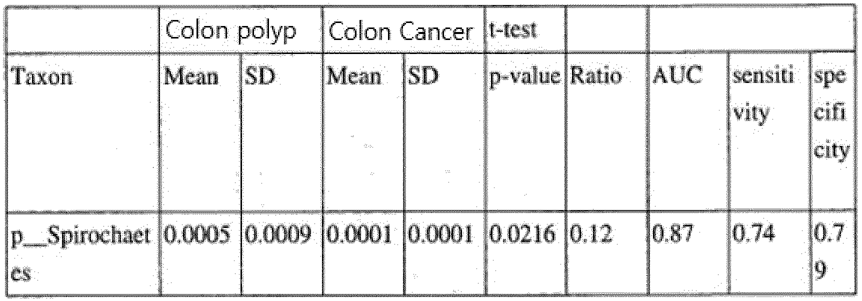

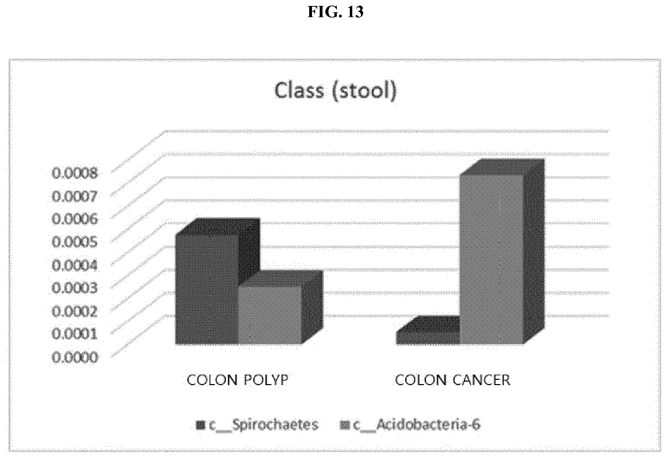

As a result of analyzing bacteria-derived extracellular vesicles in stool at a class level, a diagnostic model developed using bacteria belonging to the class *Spirochaetes* and the class *Acidobacteria-6* as a biomarker exhibited significant diagnostic performance for colon cancer (see Table 13 and FIG. 13).

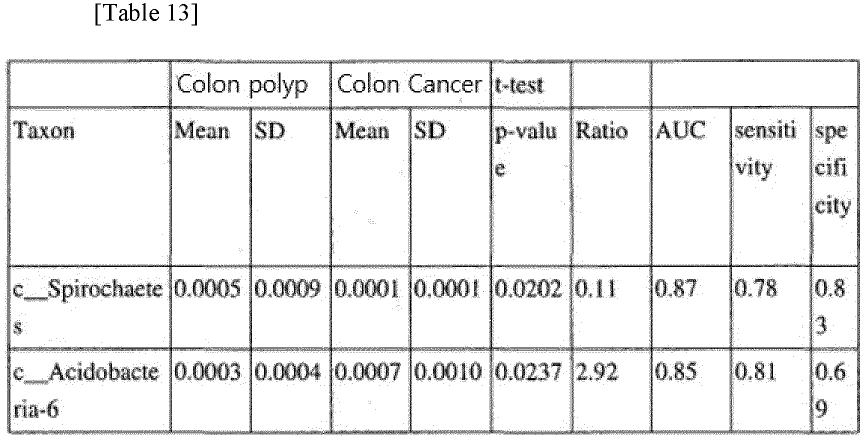

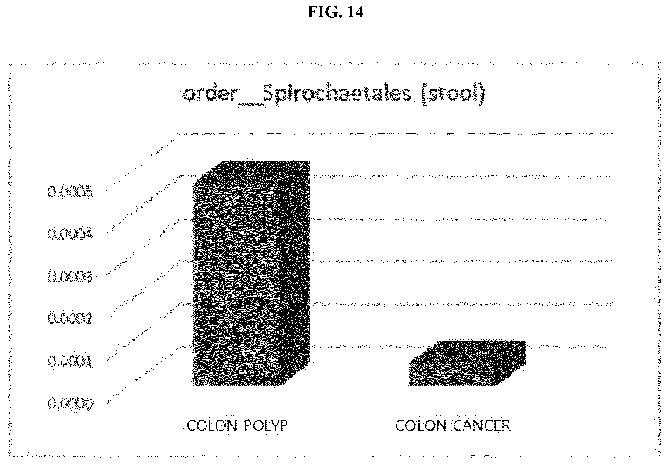

As a result of analyzing bacteria-derived extracellular vesicles in stool at an order level, a diagnostic model developed using bacteria belonging to the order *Spirochaetales* as a biomarker exhibited significant diagnostic performance for colon cancer (see Table 14 and FIG. 14).

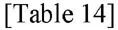

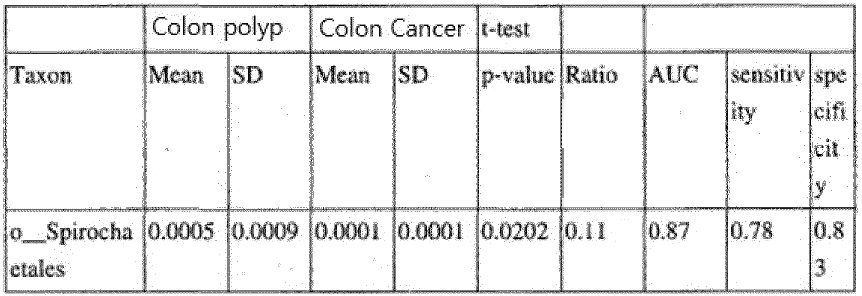

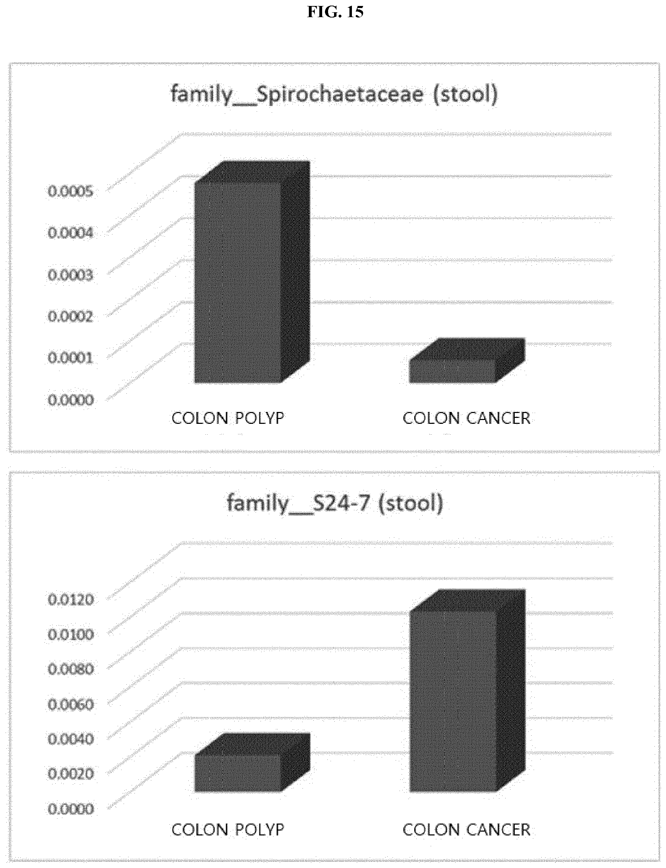

As a result of analyzing bacteria-derived extracellular vesicles in stool at a family level, a diagnostic model developed using bacteria belonging to the family *Spirochaetaceae* and the family S24-7 as a biomarker exhibited significant diagnostic performance for colon cancer (see Table 15 and FIG. 15).

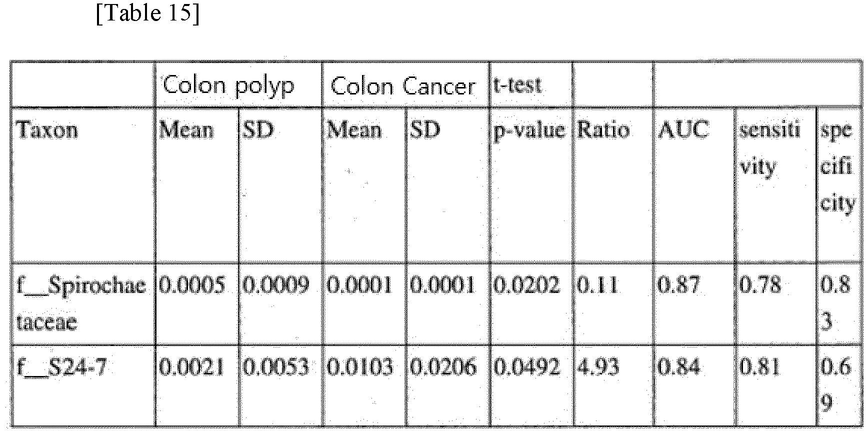

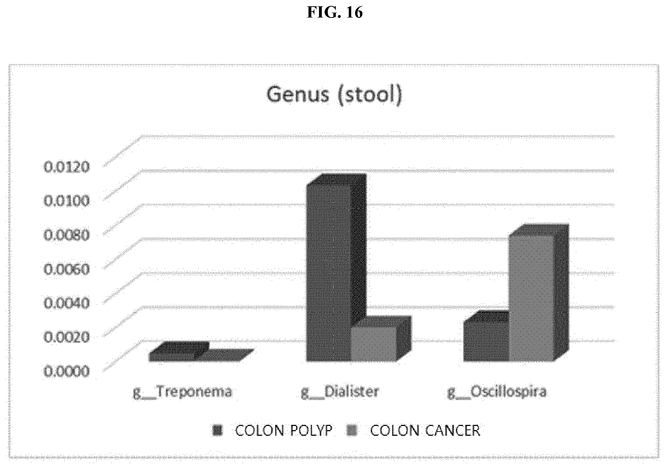

As a result of analyzing bacteria-derived extracellular vesicles in stool at a genus level, a diagnostic model developed using bacteria belonging to the genus *Treponema,* the genus *Dialister,* and the genus *Oscillospira* as a biomarker exhibited significant diagnostic performance for colon cancer (see Table 16 and FIG. 16).

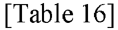

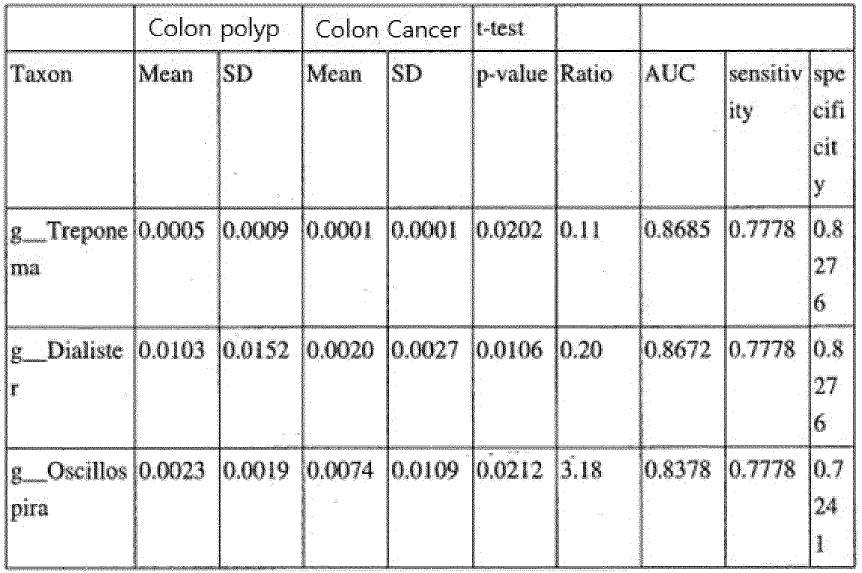

### Example 7. Colon Cancer Diagnostic Model Based on Metagenomic Analysis of Bacteria-Derived EVs Isolated from Urine of Colon Polyp Patients and Colon Cancer Patients

Extracellular vesicles were isolated from urine samples of 26 colon cancer patients and 38 colon polyp patients, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

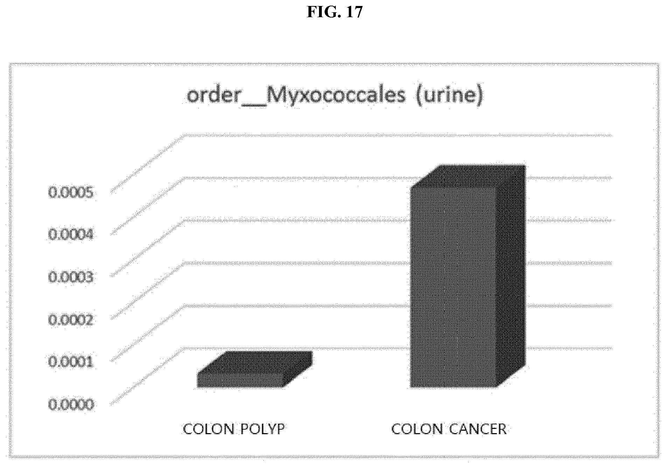

As a result of analyzing bacteria-derived extracellular vesicles in urine at an order level, a diagnostic model developed using bacteria belonging to the order *Myxococcales* exhibited significant diagnostic performance for colon cancer (see Table 17 and FIG. 17).

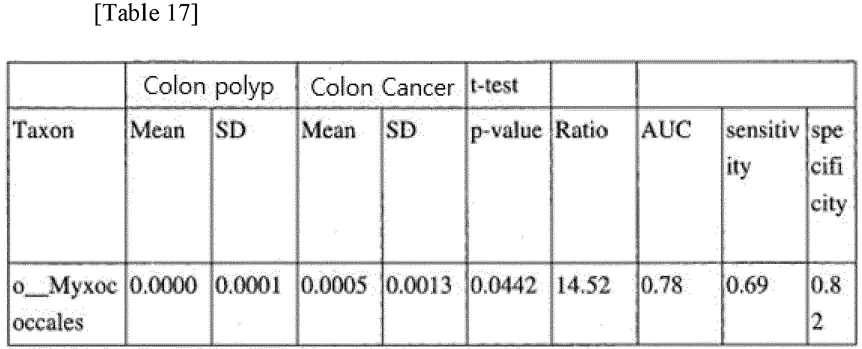

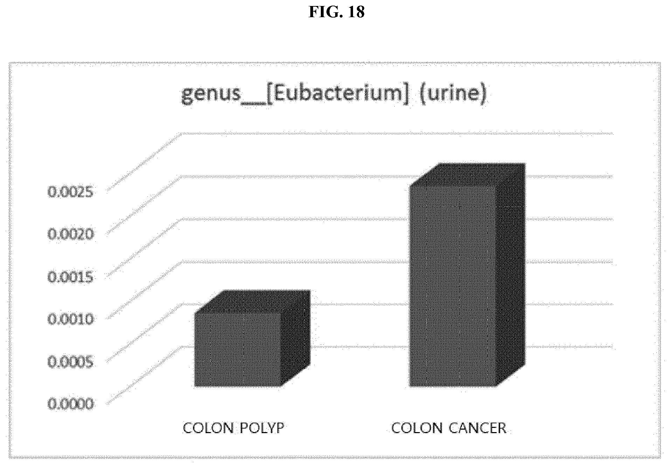

As a result of analyzing bacteria-derived extracellular vesicles in urine at a genus level, a diagnostic model developed using bacteria belonging to the genus *Eubacterium* exhibited significant diagnostic performance for colon cancer (see Table 18 and FIG. 18).

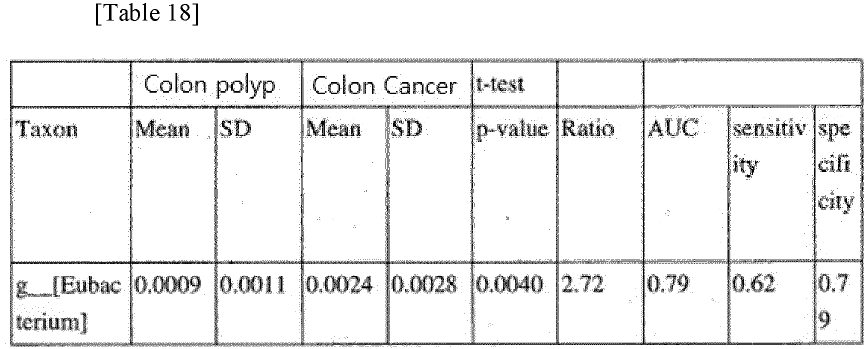

### Example 8. Colon Polyp Diagnostic Model Based on Metagenomic Analysis of Bacteria-Derived EVs Isolated from Stool of Normal Individuals and Colon Polyp Patients

Extracellular vesicles were isolated from stool samples of 27 colon cancer patients and 358 normal individuals, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

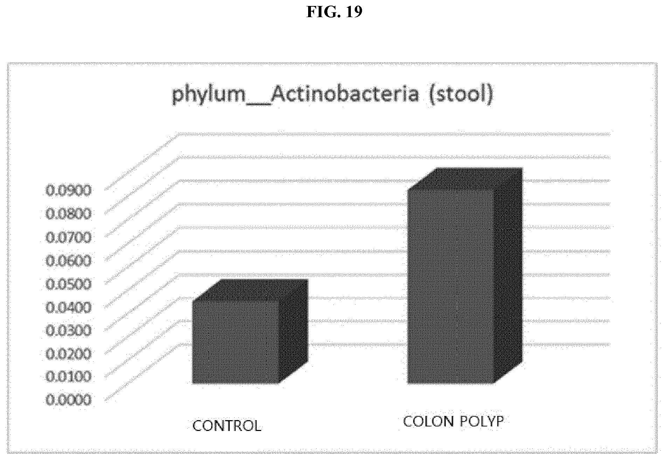

As a result of analyzing bacteria-derived extracellular vesicles in stool at a phylum level, a diagnostic model developed using bacteria belonging to the phylum *Actinobacteria* exhibited significant diagnostic performance for colon polyp (see Table 19 and FIG. 19).

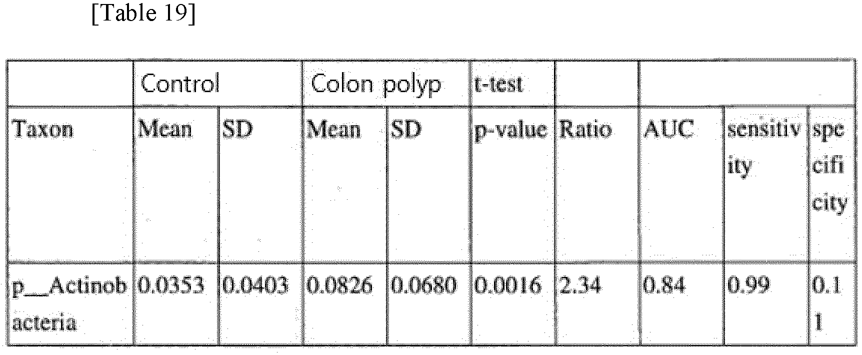

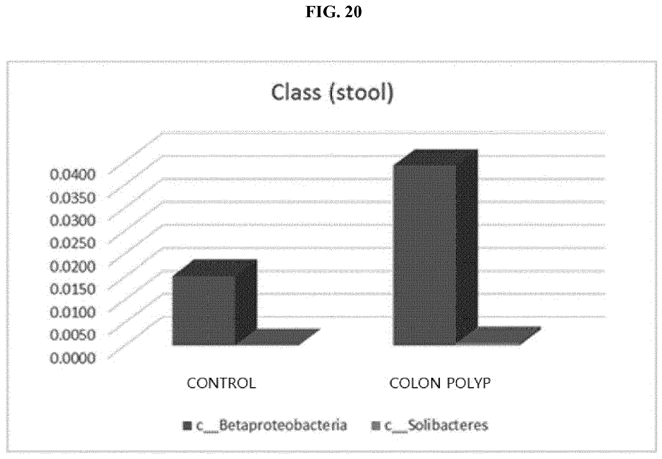

As a result of analyzing bacteria-derived extracellular vesicles in stool at a class level, a diagnostic model developed using bacteria belonging to the class *Betaproteobacteria* and the class *Solibacteres* exhibited significant diagnostic performance for colon polyp (see Table 20 and FIG. 20).

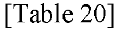

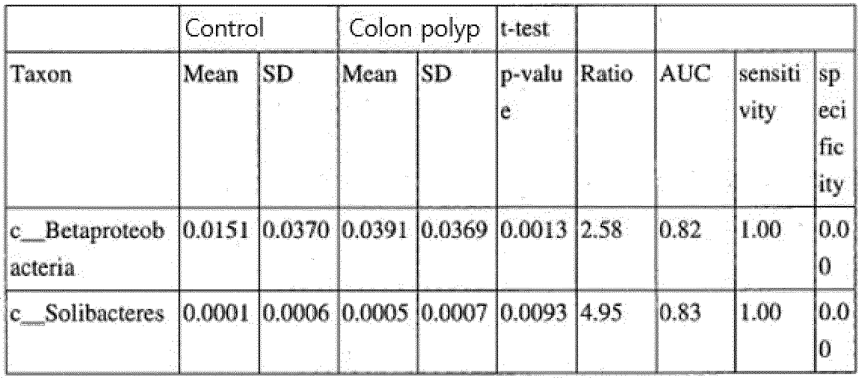

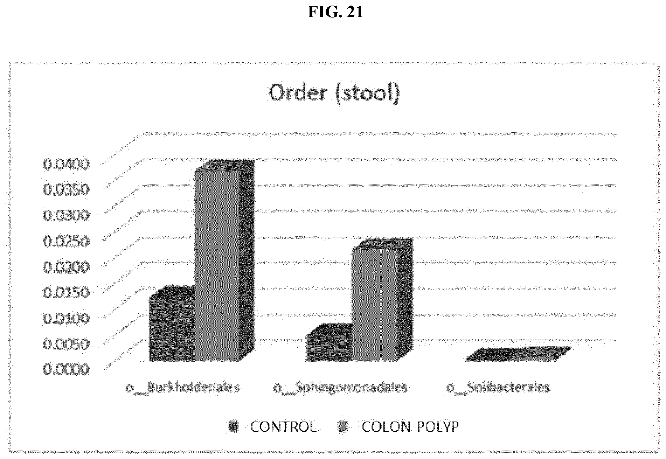

As a result of analyzing bacteria-derived extracellular vesicles in stool at an order level, a diagnostic model developed using bacteria belonging to the order *Burkholderiales,* the order *Sphingomonadales,* and the order *Solibacterales* exhibited significant diagnostic performance for colon polyp (see Table 21 and FIG. 21).

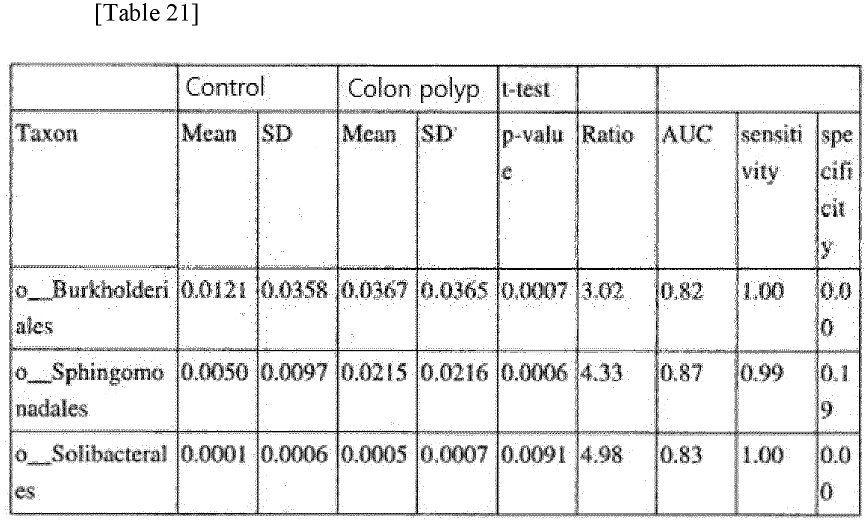

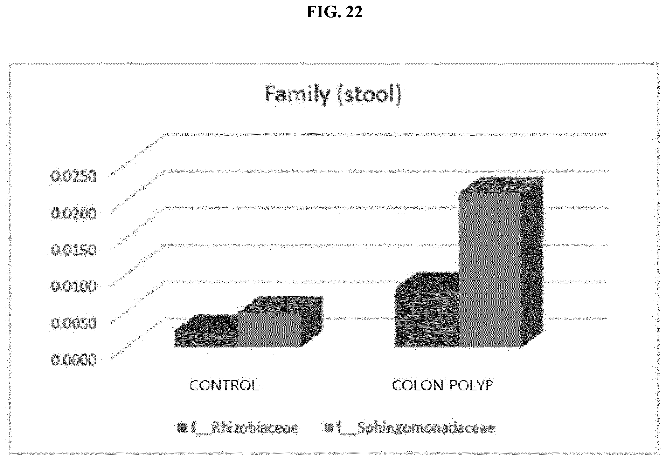

As a result of analyzing bacteria-derived extracellular vesicles in stool at a family level, a diagnostic model developed using bacteria belonging to the family *Rhizobiaceae* and the family *Sphingomonadaceae* exhibited significant diagnostic performance for colon polyp (see Table 22 and FIG. 22).

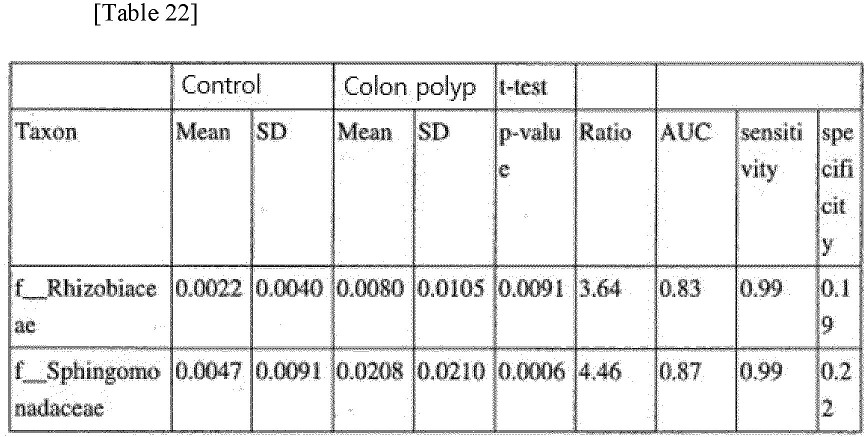

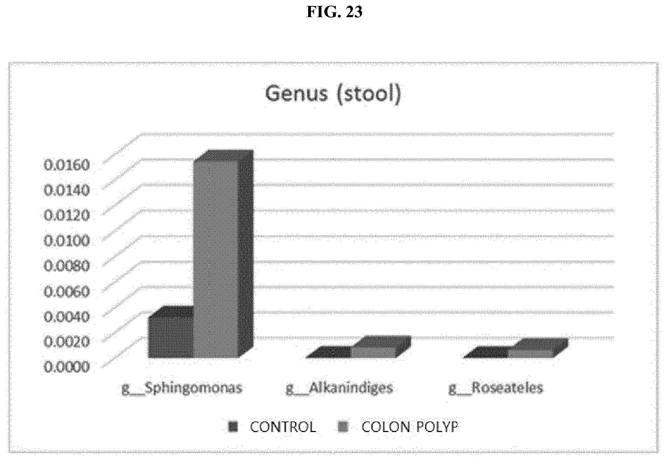

As a result of analyzing bacteria-derived extracellular vesicles in stool at a genus level, a diagnostic model developed using bacteria belonging to the genus *Sphingomonas,* the genus *Alkanindiges,* and the genus *Roseateles* exhibited significant diagnostic performance for colon polyp (see Table 23 and FIG. 23).

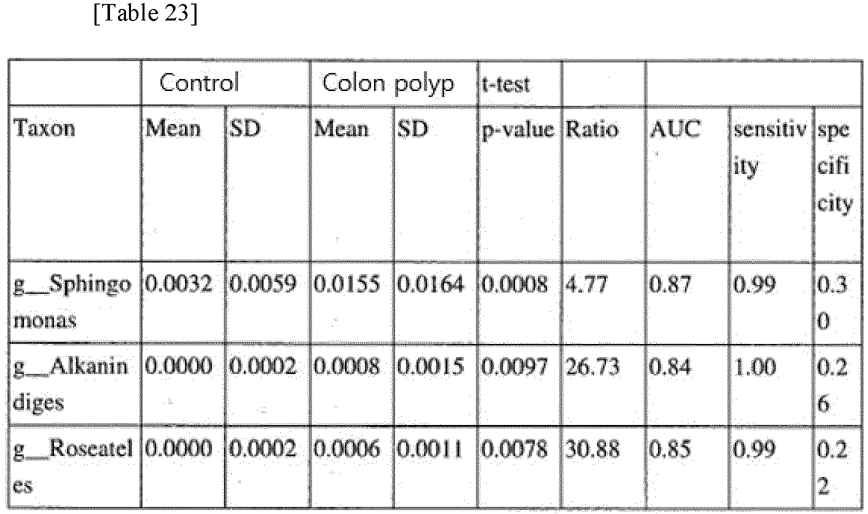

### Example 9. Colon Polyp Diagnostic Model Based on Metagenomic Analysis of Bacteria-Derived EVs Isolated from Urine of Normal Individuals and Colon Polyp Patients

Extracellular vesicles were isolated from urine samples of 38 colon cancer patients and 38 normal individuals, and then metagenomic sequencing was performed thereon using the method of Example 3. For the development of a diagnostic model, first, a strain exhibiting a p value of less than 0.05 between two groups in a t-test and a difference of two-fold or more between two groups was selected, and then an area under curve (AUC), sensitivity, and specificity, which are diagnostic performance indexes, were calculated by logistic regression analysis.

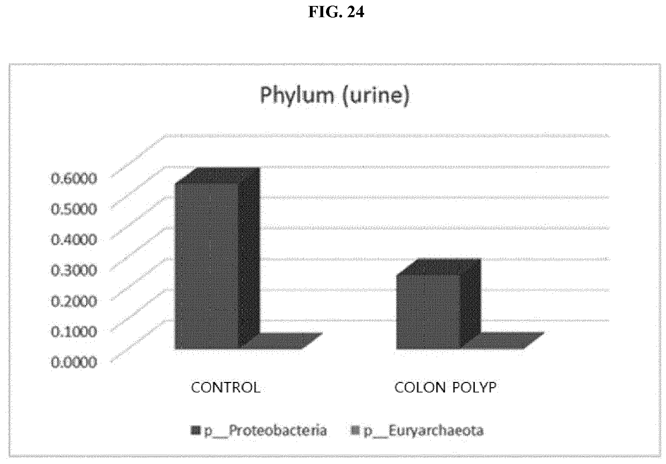

As a result of analyzing bacteria-derived extracellular vesicles in urine at a phylum level, a diagnostic model developed using bacteria belonging to the phylum *Proteobacteria* and the phylum *Euryarchaeota* exhibited significant diagnostic performance for colon polyp (see Table 24 and FIG. 24).

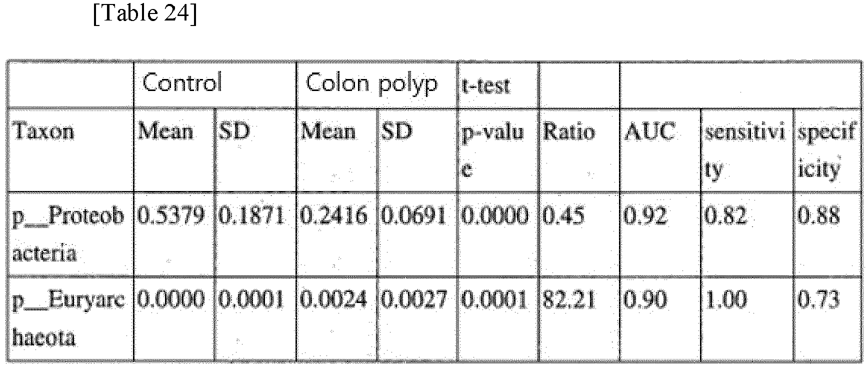

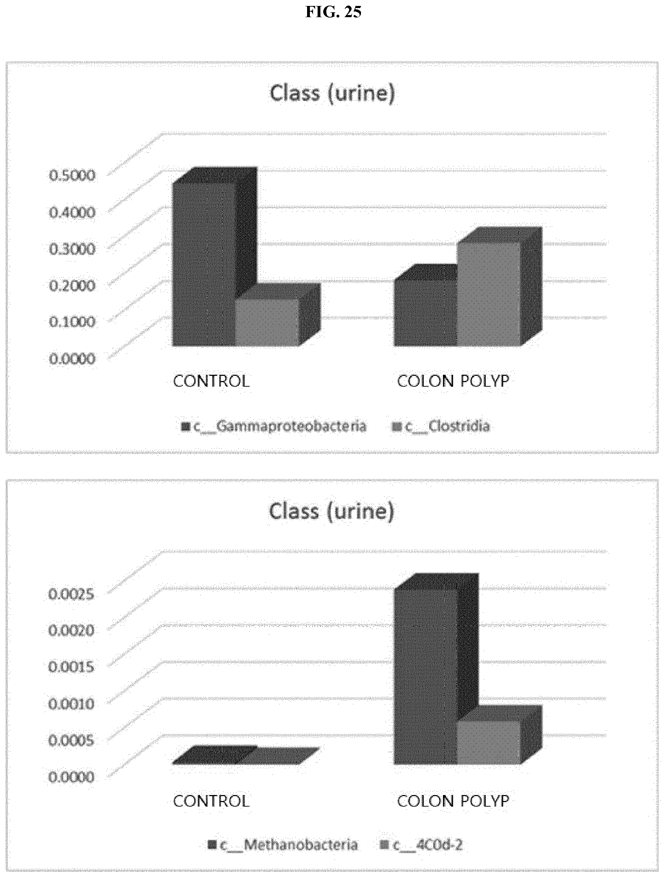

As a result of analyzing bacteria-derived extracellular vesicles in urine at a class level, a diagnostic model developed using bacteria belonging to the class *Gammaproteobacteria,* the class *Clostridia,* the class *Methanobacteria,* and the class 4C0d-2 exhibited significant diagnostic performance for colon polyp (see Table 25 and FIG. 25).

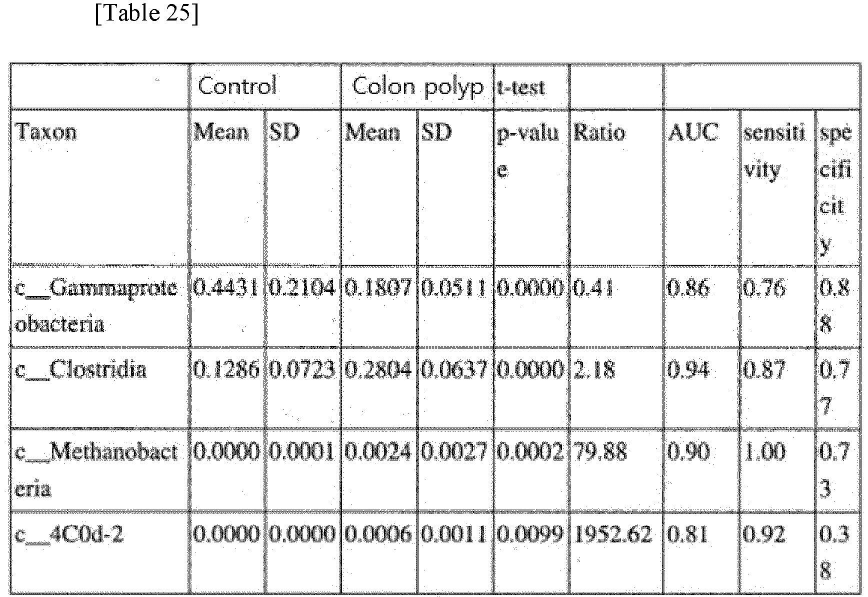

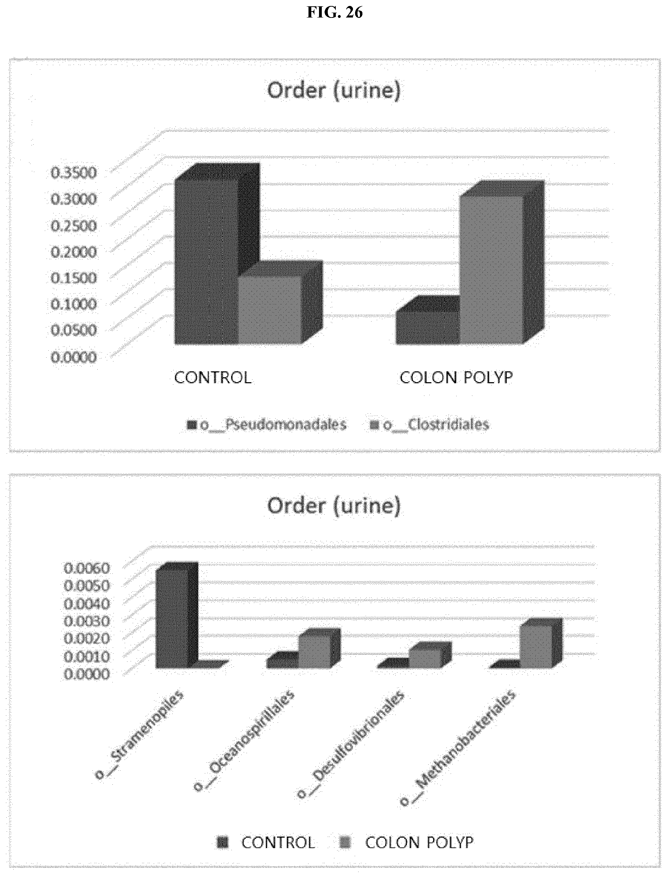

As a result of analyzing bacteria-derived extracellular vesicles in urine at an order level, a diagnostic model developed using bacteria belonging to the order *Stramenopiles,* the order *Pseudomonadales,* the order *Clostridiales,* the order *Oceanospirillales,* the order *Desulfovibrionales,* and the order *Methanobacteriales* exhibited significant diagnostic performance for colon polyp (see Table 26 and FIG. 26).

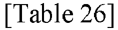

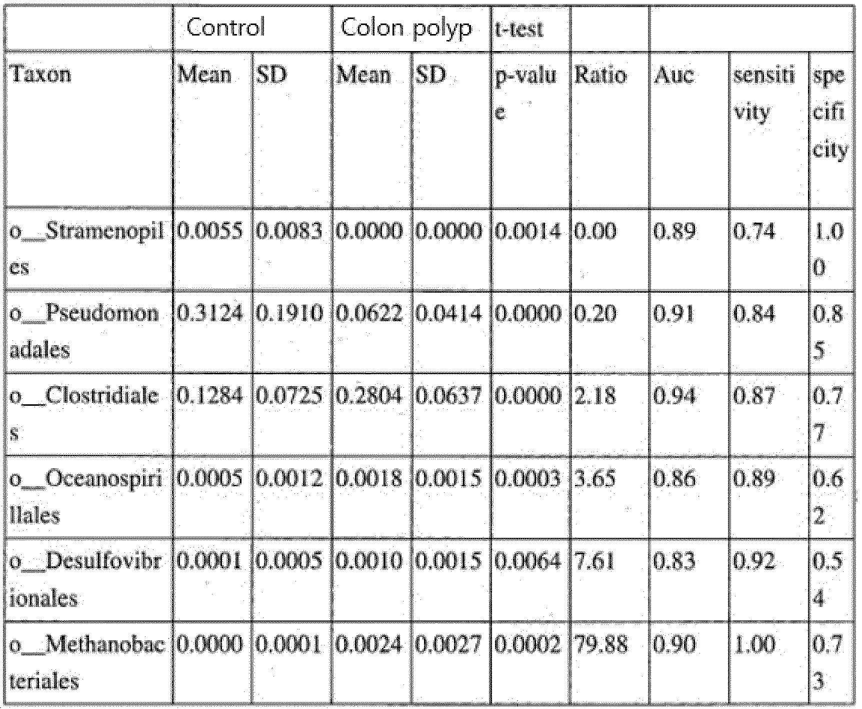

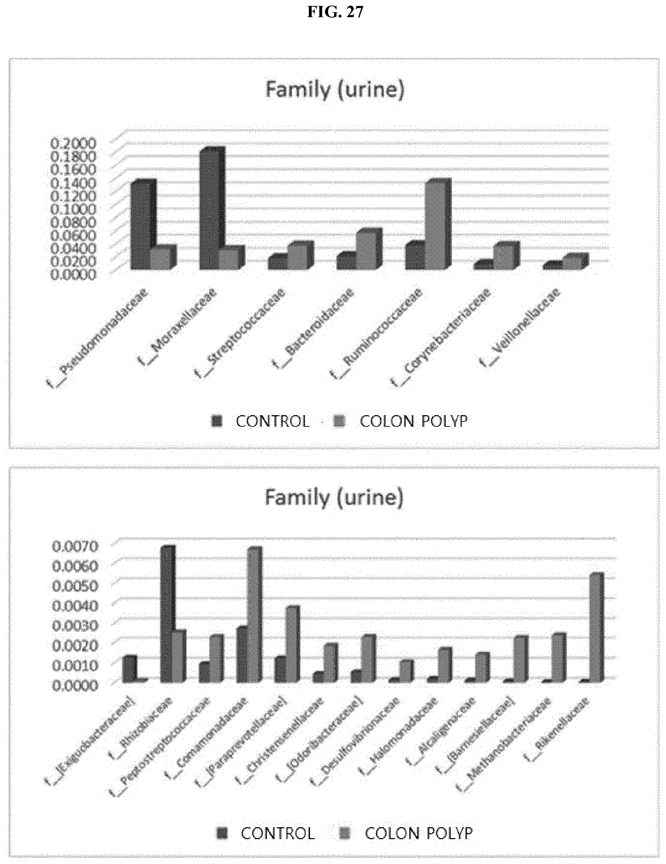

As a result of analyzing bacteria-derived extracellular vesicles in urine at a family level, a diagnostic model developed using bacteria belonging to the family *Exiguobacteraceae,* the family *Moraxellaceae,* the family *Pseudomonadaceae,* the family *Rhizobiaceae,* the family *Streptococcaceae,* the family *Peptostreptococcaceae,* the family *Comamonadaceae,* the family *Veillonellaceae,* the family *Bacteroidaceae,* the family *Paraprevotellaceae,* the family *Ruminococcaceae,* the family *Corynebacteriaceae,* the family *Christensenellaceae,* the family *Odoribacteraceae,* the family *Desulfovibrionaceae,* the family *Halomonadaceae,* the family *Alcaligenaceae,* the family *Barnesiellaceae,* the family *Methanobacteriaceae,* and the family *Rikenellaceae* exhibited significant diagnostic performance for colon polyp (see Table 27 and FIG. 27).

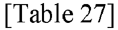

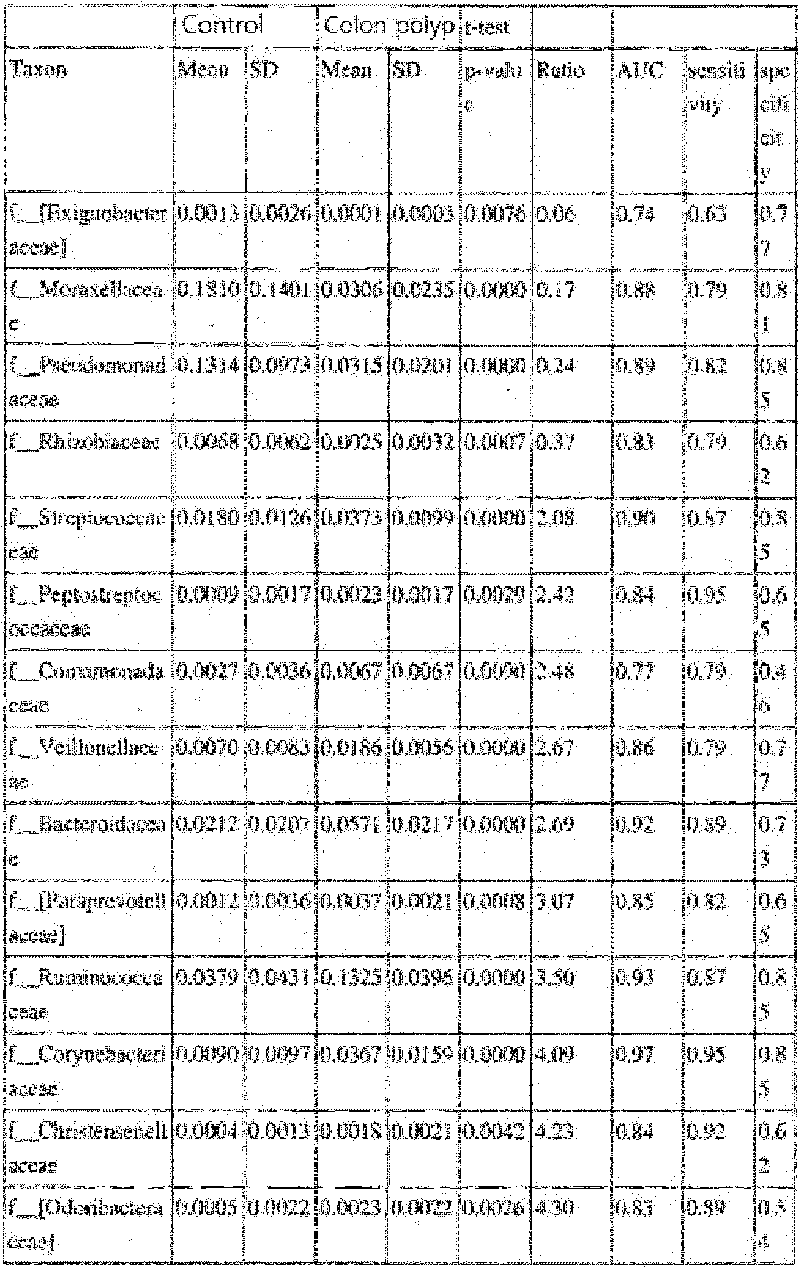

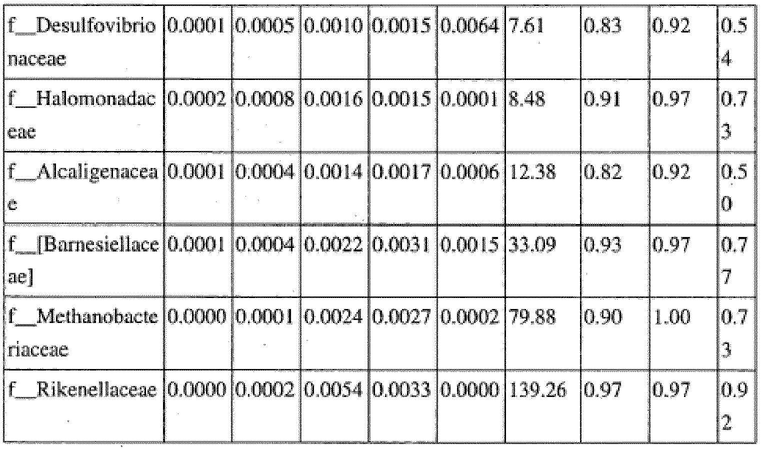

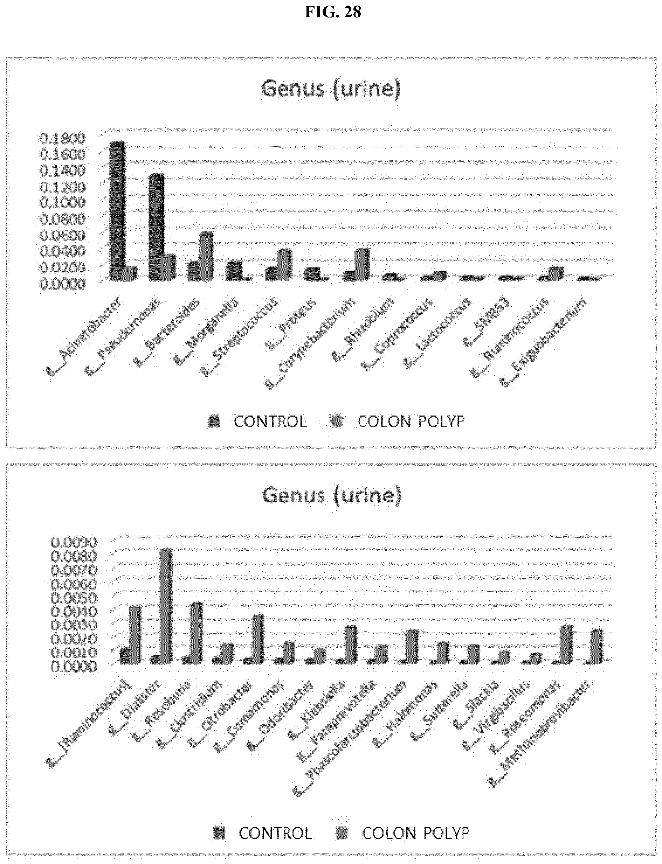

As a result of analyzing bacteria-derived extracellular vesicles in urine at a genus level, a diagnostic model developed using bacteria belonging to the genus *Rhizobium,* the genus *Morganella,* the genus *Proteus,* the genus *Exiguobacterium,* the genus *Acinetobacter,* the genus *Pseudomonas,* the genus SMB53, the genus *Lactococcus,* the genus *Coprococcus,* the genus *Streptococcus,* the genus *Bacteroides,* the genus *Ruminococcus,* the genus *Corynebacterium,* the genus *Odoribacter,* the genus *Clostridium,* the genus *Comamonas,* the genus *Paraprevotella,* the genus *Roseburia,* the genus *Citrobacter,* the genus *Klebsiella,* the genus *Virgibacillus,* the genus *Slackia,* the genus *Dialister,* the genus *Phascolarctobacterium,* the genus *Sutterella,* the genus *Halomonas,* the genus *Roseomonas,* and the genus *Methanobrevibacter* exhibited significant diagnostic performance for colon polyp (see Table 28 and FIG. 28).

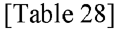

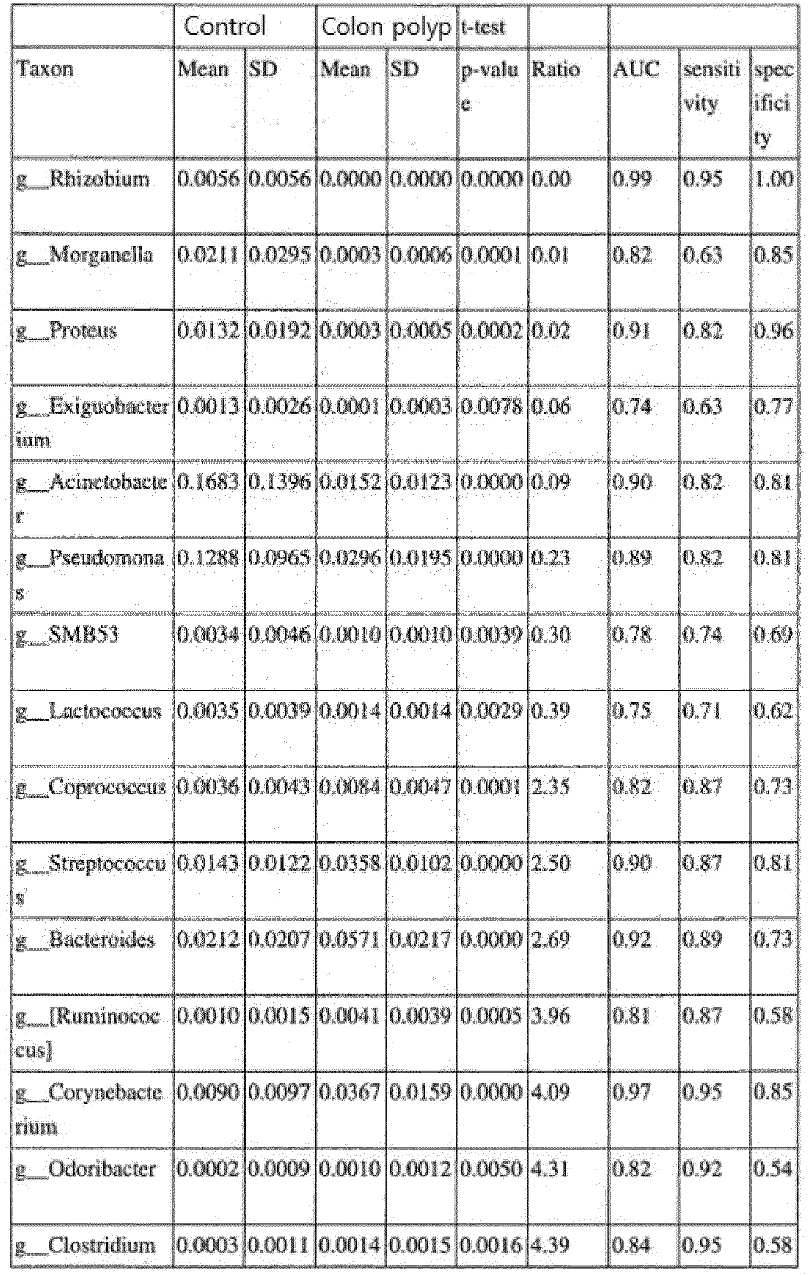

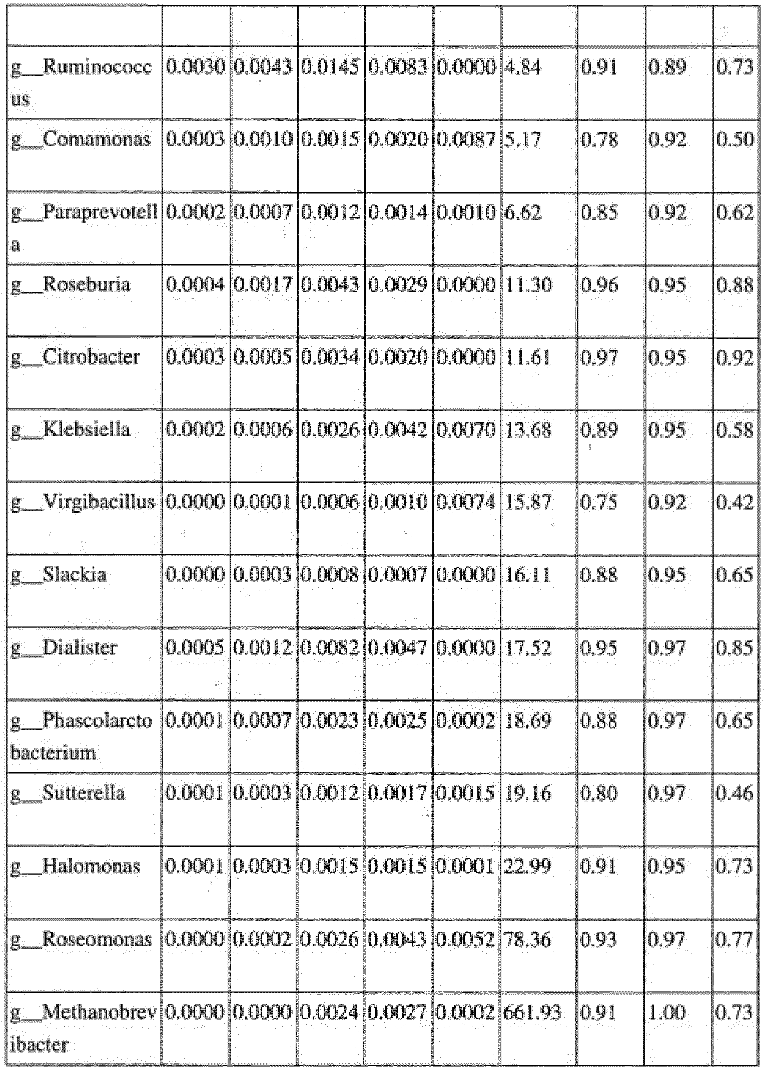

### [Industrial Applicability]

A method of diagnosing colon tumors through bacterial metagenomic analysis can be used to predict a risk for colon tumors such as a colon polyp, colon cancer, and the like and diagnose colon tumors by analyzing an increase or decrease in content of extracellular vesicles derived from specific bacteria through bacterial metagenomic analysis using a subject-derived sample. Extracellular vesicles secreted from bacteria present in the environment are absorbed into the human body, and thus may directly affect the occurrence of inflammation and cancer, and it is difficult to diagnose a colon polyp and colon cancer early before symptoms occur, and thus effective treatment thereof is difficult. Thus a risk for colon tumors such as a colon polyp, colon cancer, and the like can be predicted through metagenomic analysis of bacteria or bacteria-derived extracellular vesicles using a human bodyderived sample, and thus the onset of colon tumors can be delayed or colon tumors can be prevented through appropriate management by early diagnosis and prediction of a risk group for a colon tumor, and, even after a colon tumor occurs, early diagnosis for a colon tumor can be implemented, thereby lowering an incidence rate of a colon tumor and increasing therapeutic effects. In addition, patients diagnosed with a colon polyp or colon cancer are able to avoid exposure to causative factors predicted by the bacterial metagenomic analysis whereby the progression of a colon polyp and colon cancer can be ameliorated, or recurrence thereof can be prevented.

## Claims

1. A method of diagnosing colon tumor, the method comprising the following processes:
(a) extracting DNA from extracellular vesicles isolated from a subject sample;
(b) performing polymerase chain reaction (PCR) on the extracted DNA using a pair of primers having SEQ ID NO: 1 and SEQ ID NO: 2; and
(c) comparing an increase or decrease in content of bacteria-derived extracellular vesicles to a normal individual-derived sample through sequencing of a product of the PCR,
wherein, in process (c), colon cancer is diagnosed, wherein the comparing comprises comparing an increase or decrease in content of:
extracellular vesicles derived from one or more bacteria selected from the group consisting of
the phylum *Tenericutes,* the phylum *Actinobacteria,* the phylum *Acidobacteria,* the phylum *Armatimonadetes,* the phylum *Planctomycetes,* the phylum *Fusobacteria,* the phylum *Proteobacteria,* the phylum *Euryarchaeota;*
the class *Deferribacteres,* the class *Mollicutes,* the class *Bacilli,* the class *Alphaproteobacteria,* the class *Saprospirae,* the class *Fimbriimonadia,* the class *Acidobacteria-6,* the class *Solibacteres,* the class *Coriobacteriia,* the class *Oscillatoriophycideae,* the class *Fusobacteriia,* the class *Gammaproteobacteria,* the class *Clostridia,* the class *Methanobacteria*;
the order *Deferribacterales,* the order *Turicibacterales,* the order *Oceanospirillales,* the order *Rhizobiales,* the order *Lactobacillales,* the order *Rhodobacterales,* the order *Saprospirales,* the order *Sphingomonadales,* the order *Fimbriimonadales,* the order *Solibacterales,* the order *Coriobacteriales,* the order *Chroococcales,* the order *Fusobacteriales,* the order *Bdellovibrionales,* the order *Desulfobacterales,* the order *Stramenopiles,* the order *Pseudomonadales,* the order *Desulfovibrionales,* the order *Methanobacteriales;*
the family *Peptococcaceae,* the family *Deferribacteraceae,* the family *Turicibacteraceae,* the family *Halomonadaceae,* the family *Clostridiaceae,* the family *Prevotellaceae,* the family *Peptostreptococcaceae,* the family *Rhodobacteraceae,* the family *Nocardioidaceae,* the family *Sphingomonadaceae,* the family *Bartonellaceae,* the family *Cellulomonadaceae,* the family *Lactobacillaceae,* the family *Rhizobiaceae,* the family *Fimbriimonadaceae,* the family *Dermacoccaceae,* the family *Leptotrichiaceae,* the family *Coriobacteriaceae,* the family *Xenococcaceae,* the family *Aeromonadaceae,* the family *Geodermatophilaceae,* the family *Bdellovibrionaceae,* the family *Moraxellaceae,* the family *Pseudomonadaceae,* the family *Streptococcaceae,* the family *Veillonellaceae,* the family *Bacteroidaceae,* the family *Aerococcaceae,* the family *Comamonadaceae,* the family *Paraprevotellaceae,* the family *Christensenellaceae,* the family *Ruminococcaceae,* the family *Corynebacteriaceae,* the family *Gordoniaceae,* the *family Mycobacteriaceae,* the family *Desulfovibrionaceae,* the family *Alcaligenaceae,* the family *Barnesiellaceae,* the family *Methanobacteriaceae,* the family *Rikenellaceae;*
the genus *Catenibacterium,* the genus *Mucispirillum,* the genus *Eubacterium,* the genus *Turicibacter,* the genus *Alloiococcus,* the genus *Halomonas,* the genus *Prevotella,* the genus *Dialister,* the genus *Anaerostipes,* the genus *Faecalibacterium,* the genus *Blautia,* the genus *Capnocytophaga,* the genus *Sphingomonas,* the genus *Lactobacillus,* the genus *Fimbriimonas,* the genus *Dermacoccus,* the genus *Achromobacter,* the genus *Novosphingobium,* the genus *Sneathia,* the genus *Agrobacterium,* the genus *Blastomonas,* the genus *Bdellovibrio,* the genus *Alkanindiges,* the genus *Roseateles,* the genus *Shuttleworthia,* the genus *Rhizobium,* the genus *Morganella,* the genus *Acinetobacter,* the genus *Pseudomonas,* the genus *Enterococcus,* the genus *Lactococcus,* the genus *Coprococcus,* the genus *Dorea,* the genus *Streptococcus,* the genus *Lachnospira,* the genus *Ruminococcus,* the genus *Corynebacterium,* the genus *Comamonas,* the genus *Gordonia,* the genus *Paraprevotella,* the genus *Mycobacterium,* the genus *Roseburia,* the genus *Slackia,* the genus *Escherichia,* the genus *Phascolarctobacterium,* the genus *Sutterella,* the genus *Virgibacillus,* the genus *Eggerthella,* the genus *Citrobacter,* the genus *Roseomonas,* the genus *Serratia,* the genus *Methanobrevibacter,* and the genus *Bilophila;* or
wherein, in process (c), colon polyp is diagnosed, wherein the comparing comprises comparing an increase or decrease in content of:
extracellular vesicles derived from one or more bacteria selected from the group consisting of
the phylum *Proteobacteria,* the phylum *Euryarchaeota;*
the class *Gammaproteobacteria,* the class *Clostridia,* the class *Methanobacteria*;
the order *Stramenopiles,* the order *Pseudomonadales,* the order *Oceanospirillales,* the order *Desulfovibrionales,* the order *Methanobacteriales*;
the family *Rhizobiaceae,* the family *Exiguobacteraceae,* the family *Moraxellaceae,* the family *Pseudomonadaceae,* the family *Streptococcaceae,* the family *Peptostreptococcaceae,* the family *Comamonadaceae,* the family *Veillonellaceae,* the family *Bacteroidaceae,* the family *Paraprevotellaceae,* the family *Ruminococcaceae,* the family *Corynebacteriaceae,* the family *Christensenellaceae,* the family *Odoribacteraceae,* the family *Desulfovibrionaceae,* the family *Halomonadaceae,* the family *Alcaligenaceae,* the family *Barnesiellaceae,* the family *Methanobacteriaceae,* the family *Rikenellaceae*;
the genus *Rhizobium,* the genus *Morganella,* the genus *Exiguobacterium,* the genus *Acinetobacter,* the genus *Pseudomonas,* the genus *Lactococcus,* the genus *Coprococcus,* the genus *Streptococcus,* the genus *Ruminococcus,* the genus *Corynebacterium,* the genus *Odoribacter,* the genus *Clostridium,* the genus *Comamonas,* the genus *Paraprevotella,* the genus *Roseburia,* the genus *Citrobacter,* the genus *Klebsiella,* the genus *Virgibacillus,* the genus *Slackia,* the genus *Dialister,* the genus *Phascolarctobacterium,* the genus *Sutterella,* the genus *Halomonas,* the genus *Roseomonas,* and the genus *Methanobrevibacter.*

2. The method of claim 1, wherein
in process (c), colon cancer is diagnosed, wherein the comparing comprises comparing an increase or decrease in content of:
extracellular vesicles derived from one or more bacteria selected from the group consisting of the genus *Catenibacterium,* the genus *Mucispirillum,* the genus *Eubacterium,* the genus *Turicibacter,* the genus *Alloiococcus,* the genus *Halomonas,* the genus *Prevotella,* the genus *Dialister,* the genus *Anaerostipes,* the genus *Faecalibacterium,* the genus *Blautia,* the genus *Capnocytophaga,* the genus *Sphingomonas,* the genus *Lactobacillus,* the genus *Fimbriimonas,* the genus *Dermacoccus,* the genus *Achromobacter,* the genus *Novosphingobium,* the genus *Sneathia,* the genus *Agrobacterium,* the genus *Blastomonas,* the genus *Bdellovibrio,* the genus *Alkanindiges,* the genus *Roseateles,* the genus *Shuttleworthia,* the genus *Rhizobium,* the genus *Morganella,* the genus *Acinetobacter,* the genus *Pseudomonas,* the genus *Enterococcus,* the genus *Lactococcus,* the genus *Coprococcus,* the genus *Dorea,* the genus *Streptococcus,* the genus *Lachnospira,* the genus *Ruminococcus,* the genus *Corynebacterium,* the genus *Comamonas,* the genus *Gordonia,* the genus *Paraprevotella,* the genus *Mycobacterium,* the genus *Roseburia,* the genus *Slackia,* the genus *Escherichia,* the genus *Phascolarctobacterium,* the genus *Sutterella,* the genus *Virgibacillus,* the genus *Eggerthella,* the genus *Citrobacter,* the genus *Roseomonas,* the genus *Serratia,* the genus *Methanobrevibacter,* and the genus *Bilophila;* or
in process (c), colon polyp is diagnosed, wherein the comparing comprises comparing an increase or decrease in content of:
extracellular vesicles derived from one or more bacteria selected from the group consisting of the genus *Rhizobium,* the genus *Morganella,* the genus *Exiguobacterium,* the genus *Acinetobacter,* the genus *Pseudomonas,* the genus *Lactococcus,* the genus *Coprococcus,* the genus *Streptococcus,* the genus *Ruminococcus,* the genus *Corynebacterium,* the genus *Odoribacter,* the genus *Clostridium,* the genus *Comamonas,* the genus *Paraprevotella,* the genus *Roseburia,* the genus *Citrobacter,* the genus *Klebsiella,* the genus *Virgibacillus,* the genus *Slackia,* the genus *Dialister,* the genus *Phascolarctobacterium,* the genus *Sutterella,* the genus *Halomonas,* the genus *Roseomonas,* and the genus *Methanobrevibacter.*

3. The method of claim 1 or claim 2, wherein the subject sample is stool or urine.

## Patentansprüche

1. Verfahren zum Diagnostizieren von Kolontumoren, wobei das Verfahren die folgenden Prozesse umfasst:
(a) Extrahieren von DNA aus extrazellulären Vesikeln, die aus einer Patientenprobe isoliert wurden;
(b) Durchführen einer Polymerase-Kettenreaktion (PCR) an der extrahierten DNA unter Verwendung eines Primer-Paares, das SEQ ID Nr: 1 und SEQ ID Nr: 2 aufweist; und
(c) Vergleichen einer Zunahme oder Abnahme des Gehalts an von Bakterien stammenden extrazellulären Vesikeln mit einer von einem normalen Individuum stammenden Probe durch Sequenzieren eines Produkts der PCR,
wobei in Prozess (c) Kolonkrebs diagnostiziert wird, wobei das Vergleichen ein Vergleichen einer Zunahme oder Abnahme umfasst des Gehalts an:
extrazellulären Vesikeln, die von einer oder mehreren Bakterien stammen, ausgewählt aus der Gruppe bestehend aus
dem Stamm Tenericutes, dem Stamm Actinobacteria, dem Stamm Acidobacteria, dem Stamm Armatimonadetes, dem Stamm Planctomycetes, dem Stamm Fusobacteria, dem Stamm Proteobacteria, dem Stamm Euryarchaeota;
der Klasse Deferribacteres, der Klasse Mollicutes, der Klasse Bacilli, der Klasse Alphaproteobacteria, der Klasse Saprospirae, der Klasse Fimbriimonadia, der Klasse Acidobacteria-6, der Klasse Solibacteres, der Klasse Coriobacteriia, der Klasse Oscillatoriophycideae, der Klasse Fusobacteriia, der Klasse Gammaproteobacteria, der Klasse Clostridia, der Klasse Methanobacteria;
der Ordnung Deferribacterales, der Ordnung Turicibacterales, der Ordnung Oceanospirillales, der Ordnung Rhizobiales, der Ordnung Lactobacillales, der Ordnung Rhodobacterales, der Ordnung Saprospirales, der Ordnung Sphingomonadales, der Ordnung Fimbriimonadales, der Ordnung Solibacterales, der Ordnung Coriobacteriales, der Ordnung Chroococcales, der Ordnung Fusobacteriales, der Ordnung Bdellovibrionales, der Ordnung Desulfobacterales, der Ordnung Stramenopiles, der Ordnung Pseudomonadales, der Ordnung Desulfovibrionales, der Ordnung Methanobacteriales;
der Familie Peptococcaceae, der Familie Deferribacteraceae, der Familie Turicibacteraceae, der Familie Halomonadaceae, der Familie Clostridiaceae, der Familie Prevotellaceae, der Familie Peptostreptococcaceae, der Familie Rhodobacteraceae, der Familie Nocardioidaceae, der Familie Sphingomonadaceae, der Familie Bartonellaceae, der Familie Cellulomonadaceae, der Familie Lactobacillaceae, der Familie Rhizobiaceae, der Familie Fimbriimonadaceae, der Familie Dermacoccaceae, der Familie Leptotrichiaceae, der Familie Coriobacteriaceae, der Familie Xenococcaceae, der Familie Aeromonadaceae, der Familie Geodermatophilaceae, der Familie Bdellovibrionaceae, der Familie Moraxellaceae, der Familie Pseudomonadaceae, der Familie Streptococcaceae, der Familie Veillonellaceae, der Familie Bacteroidaceae, der Familie Aerococcaceae, der Familie Comamonadaceae, der Familie Paraprevotellaceae, der Familie Christensenellaceae, der Familie Ruminococcaceae, der Familie Corynebacteriaceae, der Familie Gordoniaceae, der Familie Mycobacteriaceae, der Familie Desulfovibrionaceae, der Familie Alcaligenaceae, der Familie Barnesiellaceae, der Familie Methanobacteriaceae, der Familie Rikenellaceac,
der Gattung Catenibacterium, der Gattung Mucispirillum, der Gattung Eubacterium, der Gattung Turicibacter, der Gattung Alloiococcus, der Gattung Halomonas, der Gattung Prevotella, der Gattung Dialister, der Gattung Anaerostipes, der Gattung Faecalibacterium, der Gattung Blautia, der Gattung Capnocytophaga, der Gattung Sphingomonas, der Gattung Lactobacillus, der Gattung Fimbriimonas, der Gattung Dermacoccus, der Gattung Achromobacter, der Gattung Novosphingobium, der Gattung Sneathia, der Gattung Agrobacterium, der Gattung Blastomonas, der Gattung Bdellovibrio, der Gattung Alkanindiges, der Gattung Roseateles, der Gattung Shuttleworthia, der Gattung Rhizobium, der Gattung Morganella, der Gattung Acinetobacter, der Gattung Pseudomonas, der Gattung Enterococcus, der Gattung Lactococcus, der Gattung Coprococcus, der Gattung Dorea, der Gattung Streptococcus, der Gattung Lachnospira, der Gattung Ruminococcus, der Gattung Corynebacterium, der Gattung Comamonas, der Gattung Gordonia, der Gattung Paraprevotella, der Gattung Mycobacterium, der Gattung Roseburia, der Gattung Slackia, der Gattung Escherichia, der Gattung Phascolarctobacterium, der Gattung Sutterella, der Gattung Virgibacillus, der Gattung Eggerthella, der Gattung Citrobacter, der Gattung Roseomonas, der Gattung Serratia, der Gattung Methanobrevibacter, und der Gattung Bilophila; oder
wobei in Prozess (c) Kolonpolypen diagnostiziert werden, wobei das Vergleichen ein Vergleichen einer Zunahme oder Abnahme umfasst des Gehalts an:
extrazellulären Vesikeln, die von einer oder mehreren Bakterien stammen, ausgewählt aus der Gruppe bestehend aus
dem Stamm Proteobacteria, dem Stamm Euryarchaeota;
der Klasse Gammaproteobacteria, der Klasse Clostridia, der Klasse Methanobacteria;
der Ordnung Stramenopiles, der Ordnung Pseudomonadales, der Ordnung Oceanospirillales, der Ordnung Desulfovibrionales, der Ordnung Methanobacteriales;
der Familie Rhizobiaceae, der Familie Exiguobacteraceae, der Familie Moraxellaceae, der Familie Pseudomonadaceae, der Familie Streptococcaceae, der Familie Peptostreptococcaceae, der Familie Comamonadaceae, der Familie Veillonellaceae, der Familie Bacteroidaceae, der Familie Paraprevotellaceae, der Familie Ruminococcaceae, der Familie Corynebacteriaceae, der Familie Christensenellaceae, der Familie Odoribacteraceae, der Familie Desulfovibrionaceae, der Familie Halomonadaceae, der Familie Alcaligenaceae, der Familie Bamesiellaceae, der Familie Methanobacteriaceae, der Familie Rikenellaceae;
der Gattung Rhizobium, der Gattung Morganella, der Gattung Exiguobacterium, der Gattung Acinetobacter, der Gattung Pseudomonas, der Gattung Lactococcus, der Gattung Coprococcus, der Gattung Streptococcus, der Gattung Ruminococcus, der Gattung Corynebacterium, der Gattung Odoribacter, der Gattung Clostridium, der Gattung Comamonas, der Gattung Paraprevotella, der Gattung Roseburia, der Gattung Citrobacter, der Gattung Klebsiella, der Gattung Virgibacillus, der Gattung Slackia, der Gattung Dialister, der Gattung Phascolarctobacterium, der Gattung Sutterella, der Gattung Halomonas, der Gattung Roseomonas und der Gattung Methanobrevibacter.

2. Verfahren nach Anspruch 1, wobei
in Prozess (c) Kolonkrebs diagnostiziert wird, wobei das Vergleichen ein Vergleichen einer Zunahme oder Abnahme umfasst des Gehalts an:
extrazellulären Vesikeln, die von einer oder mehreren Bakterien stammen, ausgewählt aus der Gruppe bestehend aus der Gattung Catenibacterium, der Gattung Mucispirillum, der Gattung Eubacterium, der Gattung Turicibacter, der Gattung Alloiococcus, der Gattung Halomonas, der Gattung Prevotella, der Gattung Dialister, der Gattung Anaerostipes, der Gattung Faecalibacterium, der Gattung Blautia, der Gattung Capnocytophaga, der Gattung Sphingomonas, der Gattung Lactobacillus, der Gattung Fimbriimonas, der Gattung Dermacoccus, der Gattung Achromobacter, der Gattung Novosphingobium, der Gattung Sneathia, der Gattung Agrobacterium, der Gattung Blastomonas, der Gattung Bdellovibrio, der Gattung Alkanindiges, der Gattung Roseateles, der Gattung Shuttleworthia, der Gattung Rhizobium, der Gattung Morganella, der Gattung Acinetobacter, der Gattung Pseudomonas, der Gattung Enterococcus, der Gattung Lactococcus, der Gattung Coprococcus, der Gattung Dorea, der Gattung Streptococcus, der Gattung Lachnospira, der Gattung Ruminococcus, der Gattung Corynebacterium, der Gattung Comamonas, der Gattung Gordonia, der Gattung Paraprevotella, der Gattung Mycobacterium, der Gattung Roseburia, der Gattung Slackia, der Gattung Escherichia, der Gattung Phascolarctobacterium, der Gattung Sutterella, der Gattung Virgibacillus, der Gattung Eggerthella, der Gattung Citrobacter, der Gattung Roseomonas, der Gattung Serratia, der Gattung Methanobrevibacter, und der Gattung Bilophila; oder
in Prozess (c) Kolonpolypen diagnostiziert werden, wobei das Vergleichen ein Vergleichen einer Zunahme oder Abnahme umfasst des Gehalts an:
extrazellulären Vesikeln, die von einer oder mehreren Bakterien stammen, ausgewählt aus der Gruppe bestehend aus der Gattung Rhizobium, der Gattung Morganella, der Gattung Exiguobacterium, der Gattung Acinetobacter, der Gattung Pseudomonas, der Gattung Lactococcus, der Gattung Coprococcus, der Gattung Streptococcus, der Gattung Ruminococcus, der Gattung Corynebacterium, der Gattung Odoribacter, der Gattung Clostridium, der Gattung Comamonas, der Gattung Paraprevotella, der Gattung Roseburici, der Gattung Citrobacter, der Gattung Klebsiella, der Gattung Virgibacillus, der Gattung Slackia, der Gattung Dialister, der Gattung Phascolarctobacterium, der Gattung Sutterella, der Gattung Halomonas, der Gattung Roseomonas und der Gattung Methanobrevibacter.

3. Verfahren nach Anspruch 1 oder Anspruch 2, wobei es sich bei der Patientenprobe um Stuhl oder Urin handelt.

## Revendications

1. Procédé de diagnostic d'une tumeur au côlon, le procédé comprenant les processus suivants :
(a) l'extraction d'ADN de vésicules extracellulaires isolées à partir d'un échantillon de sujet ;
(b) la réalisation d'une amplification en chaîne par polymérase (PCR) sur l'ADN extrait en utilisant une paire d'amorces présentant la SEQ ID NO : 1 et la SEQ ID NO : 2 ; et
(c) la comparaison d'une augmentation ou d'une diminution de la teneur en vésicules extracellulaires dérivées de bactéries à celle d'un échantillon dérivé d'un individu normal par séquençage d'un produit de la PCR,
dans lequel, dans le processus (c), un cancer du côlon est diagnostiqué, dans lequel la comparaison comprend la comparaison d'une augmentation ou d'une diminution de la teneur en :
vésicules extracellulaires dérivées d'une ou plusieurs bactéries sélectionnées dans le groupe consistant en
le phylum Tenericutes, le phylum Actinobacteria, le phylum Acidobacteria, le phylum Armatimonadetes, le phylum Planctomycetes, le phylum Fusobacteria, le phylum Proteobacteria, le phylum Euryarchaeota ;
la classe Deferribacteres, la classe Mollicutes, la classe Bacilli, la classe Alphaproteobacteria, la classe Saprospirae, la classe Fimbriimonadia, la classe Acidobacteria-6, la classe Solibacteres, la classe Coriobacteriia, la classe Oscillatoriophycideae, la classe Fusobacteriia, la classe Gammaproteobacteria, la classe Clostridia, la classe Methanobacteria ;
l'ordre des Deferribacterales, l'ordre des Turicibacterales, l'ordre des Oceanospirillales, l'ordre des Rhizobiales, l'ordre des Lactobacillales, l'ordre des Rhodobacteriales, l'ordre des Saprospirales, l'ordre des Sphingomonadales, l'ordre des Fimbriimonadales, l'ordre des Solibacterales, l'ordre des Coriobacteriales, l'ordre des Chroococcales, l'ordre des Fusobacteriales, l'ordre des Bdellovibrionales, l'ordre des Desulfobacterales, l'ordre des Stramenopiles, l'ordre des Pseudomonadales, l'ordre des Desulfovibrionales, l'ordre des Methanobacteriales ;
la famille Peptococcaceae, la famille Deferribacteraceae, la famille Turicibacteraceae, la famille Halomonadaceae, la famille Clostridiaceae, la famille Prevotellaceae, la famille Peptostreptococcaceae, la famille Rhodobacteraceae, la famille Nocardioidaceae, la famille Sphingomonadaceae, la famille Bartonellaceae, la famille Cellulomonadaceae, la famille Lactobacillaceae, la famille Rhizobiaceae, la famille Fimbriimonadaceae, la famille Dermacoccaceae, la famille Leptotrichiaceae, la famille Coriobacteriaceae, la famille Xenococcaceae, la famille Aeromonadaceae, la famille Geodermatophilaceae, la famille Bdellovibrionaceae, la famille Moraxellaceae, la famille Pseudomonadaceae, la famille Streptococcaceae, la famille Veillonellaceae, la famille Bacteroidaceae, la famille Aerococcaceae, la famille Comamonadaceae, la famille Paraprevotellaceae, la famille Christensenellaceae, la famille Ruminococcaceae, la famille Corynebacteriaceae, la famille Gordoniaceae, la famille Mycobacteriaceae, la famille Desulfovibrionaceae, la famille Alcaligenaceae, la famille Barnesiellaceae, la famille Methanobacteriaceae, la famille Rikenellaceae ;
le genre Catenibacterium, le genre Mucispirillum, le genre Eubacterium, le genre Turicibacter, le genre Alloiococcus, le genre Halomonas, le genre Prevotella, le genre Dialister, le genre Anaerostipes, le genre Faecalibacterium, le genre Blautia, le genre Capnocytophaga, le genre Sphingomonas, le genre Lactobacillus, le genre Fimbriimonas, le genre Dermacoccus, le genre Achromobacter, le genre Novosphingobium, le genre Sneathia, le genre Agrobacterium, le genre Blastomonas, le genre Bdellovibrio, le genre Alkanindiges, le genre Roseateles, le genre Shuttleworthia, le genre Rhizobium, le genre Morganella, le genre Acinetobacter, le genre Pseudomonas, le genre Enterococcus, le genre Lactococcus, le genre Coprococcus, le genre Dorea, le genre Streptococcus, le genre Lachnospira, le genre Ruminococcus, le genre Corynebacterium, le genre Comamonas, le genre Gordonia, le genre Paraprevotella, le genre Mycobacterium, le genre Roseburia, le genre Slackia, le genre Escherichia, le genre Phascolarctobacterium, le genre Sutterella, le genre Virgibacillus, le genre Eggerthella, le genre Citrobacter, le genre Roseomonas, le genre Serratia, le genre Methanobrevibacter et le genre Bilophila ; ou
dans lequel, dans le processus (c), un polype du côlon est diagnostiqué, dans lequel la comparaison comprend la comparaison d'une augmentation ou d'une diminution de la teneur en :
vésicules extracellulaires dérivées d'une ou plusieurs bactéries sélectionnées dans le groupe consistant en
le phylum Proteobacteria, le phylum Euryarchaeota ;
la classe Gammaproteobacteria, la classe Clostridia, la classe Methanobacteria ;
l'ordre des Stramenopiles, l'ordre des Pseudomonadales, l'ordre des Oceanospirillales, l'ordre des Desulfovibrionales, l'ordre des Methanobacteriales ;
la famille Rhizobiaceae, la famille Exiguobacteraceae, la famille Moraxellaceae, la famille Pseudomonadaceae, la famille Streptococcaceae, la famille Peptostreptococcaceae, la famille Comamonadaceae, la famille Veillonellaceae, la famille Bacteroidaceae, la famille Paraprevotellaceae, la famille Ruminococcaceae, la famille Corynebacteriaceae, la famille Christensenellaceae, la famille Odoribacteraceae, la famille Desulfovibrionaceae, la famille Halomonadaceae, la famille Alcaligenaceae, la famille Barnesiellaceae, la famille Methanobacteriaceae, la famille Rikenellaceae ;
le genre Rhizobium, le genre Morganella, le genre Exiguobacterium, le genre Acinetobacter, le genre Pseudomonas, le genre Lactococcus, le genre Coprococcus, le genre Streptococcus, le genre Ruminococcus, le genre Corynebacterium, le genre Odoribacter, le genre Clostridium, le genre Comamonas, le genre Paraprevotella, le genre Roseburia, le genre Citrobacter, le genre Klebsiella, le genre Virgibacillus, le genre Slackia, le genre Dialister, le genre Phascolarctobacterium, le genre Sutterella, le genre Halomonas, le genre Roseomonas et le genre Methanobrevibacter.

2. Procédé selon la revendication 1, dans lequel
dans le processus (c), un cancer du côlon est diagnostiqué, dans lequel la comparaison comprend la comparaison d'une augmentation ou d'une diminution de la teneur en :
vésicules extracellulaires dérivées d'une ou de plusieurs bactéries sélectionnées dans le groupe consistant en le genre Catenibacterium, le genre Mucispirillum, le genre Eubacterium, le genre Turicibacter, le genre Alloiococcus, le genre Halomonas, le genre Prevotella, le genre Dialister, le genre Anaerostipes, le genre Faecalibacterium, le genre Blautia, le genre Capnocytophaga, le genre Sphingomonas, le genre Lactobacillus, le genre Fimbriimonas, le genre Dermacoccus, le genre Achromobacter, le genre Novosphingobium, le genre Sneathia, le genre Agrobacterium, le genre Blastomonas, le genre Bdellovibrio, le genre Alkanindiges, le genre Roseateles, le genre Shuttleworthia, le genre Rhizobium, le genre Morganella, le genre Acinetobacter, le genre Pseudomonas, le genre Enterococcus, le genre Lactococcus, le genre Coprococcus, le genre Dorea, le genre Streptococcus, le genre Lachnospira, le genre Ruminococcus, le genre Corynebacterium, le genre Comamonas, le genre Gordonia, le genre Paraprevotella, le genre Mycobacterium, le genre Roseburia, le genre Slackia, le genre Escherichia, le genre Phascolarctobacterium, le genre Sutterella, le genre Virgibacillus, le genre Eggerthella, le genre Citrobacter, le genre Roseomonas, le genre Serratia, le genre Methanobrevibacter et le genre Bilophila ; ou
dans le processus (c), un polype du côlon est diagnostiqué, dans lequel la comparaison comprend la comparaison d'une augmentation ou d'une diminution de la teneur en :
vésicules extracellulaires dérivées d'une ou de plusieurs bactéries sélectionnées dans le groupe consistant en le genre Rhizobium, le genre Morganella, le genre Exiguobacterium, le genre Acinetobacter, le genre Pseudomonas, le genre Lactococcus, le genre Coprococcus, le genre Streptococcus, le genre Ruminococcus, le genre Corynebacterium, le genre Odoribacter, le genre Clostridium, le genre Comamonas, le genre Paraprevotella, le genre Roseburia, le genre Citrobacter, le genre Klebsiella, le genre Virgibacillus, le genre Slackia, le genre Dialister, le genre Phascolarctobacterium, le genre Sutterella, le genre Halomonas, le genre Roseomonas et le genre Methanobrevibacter.

3. Procédé selon la revendication 1 ou la revendication 2, dans lequel l'échantillon de sujet est un échantillon de selles ou d'urine.
